# EUROPEAN PATENT SPECIFICATION

(11) **EP 1 542 691 B1**
(45) Date of publication and mention of the grant of the patent: **07.01.2009**
(21) Application number: 03758540.3
(22) Date of filing: 29.09.2003
(51) Int. Cl.: A61K 31/4985, A61K 31/5517, A61P 35/00

(54) **COMPOSITION FOR THE TREATMENT OF NASOPHARYNGEAL CARCINOMA AND USE THEREOF**
ZUBEREITUNG ZUR BEHANDLUNG DES NASOPHARYNGALEN CARCINOM UND VERWENDUNG DERSELBEN
COMPOSITION POUR TRAITER LE CANCER DU NASOPHARYNX ET PROCEDE D'UTILISATION CORRESPONDANT

(30) Priority: 27.09.2002 US 414103 P
(43) Date of publication of application: 22.06.2005
(73) Proprietor: SOCIETE DE CONSEILS DE RECHERCHES ET D'APPLICATIONS SCIENTIFIQUES S.A.S., 75016 Paris (FR); CENTRE NATIONAL DE LA RECHERCHE SCIENTIFIQUE (CNRS), 75794 Paris Cedex 16 (FR)
(72) Inventor: PREVOST, Gregoire, F-91966 Les Ulis (FR); BUSSON, Pierre, F-94805 Ville Juif (FR); VICAT, Jean-Michel, F-94805 Ville Juif (FR)
(74) Representative: Taylor, Kate Laura
(86) International application number: PCT/IB2003/004922
(87) International publication number: WO 2004/028541

(56) References cited:
- WO-A-00/39130
- WO-A-01/64197
- WO-A-97/30053
- VICAT, J.-M- ET AL.: "Apoptosis and TRAF-1 cleavage in Epstein-Barr virus-positive nasopharyngeal carcinoma cells treated with doxorubicin combined with a farnesyl-transferase inhibitor" BIOCHEMICAL PHARMACOLOGY, vol. 65, no. 3, 2003, pages 423-433, XP002273795

## Description

### BACKGROUND OF THE INVENTION

Nasopharyngeal carcinoma (NPC), a human squamous cell cancer, arises in the surface epithelium of the posterior nasopharynx. (Liao, W.C., et al., Int. J of Oncology 17: 323-328 (2000)). Undifferentiated NPC's have several remarkable features which make them unique among human epithelial malignancies. (Ablashi, D., et al., Epstein-Barr virus and Kaposi's sarcoma Herpesvirus/Human Herpesvirus 8 IARC monographs on the evaluation of carcinogenic risks to humans. IARC, Lyon, France, n°70:97.) They are rare in most countries but they occur with a high incidence in some selected areas, especially Southeast Asia and North Africa.

NPC is consistently associated with the Epstein-Barr virus (EBV) regardless of patient geographic origin. On tissue sections, NPC appears to be heavily infiltrated by non-malignant lymphocytes mostly of the T-lineage. The full length genome of EBV is contained in all malignant epithelial cells and consistently encodes several viral products which are likely to contribute to the malignant phenotype. However, there is no production of viral particles in tumor cells; in other words, NPC cells are mainly in a state of "latent" EBV-infection. Some of the latent viral products are the EBNA1 protein and two species of small untranslated RNA's, called EBER 1 and 2. Another EBV-protein called Latent Membrane Protein 1 (LMP1) is produced in about 50% of NPC's. Other viral RNA messengers and their corresponding proteins are under investigation. (Fries, K. L., et al., Identification of a novel protein encoded by the BamHI A region of the Epstein-Barr virus. J Virol, 71 : 2765-2771, 1997; Kienzle, N., et al., Epstein-Barr virus-encoded RK-BARF0 protein expression. J Virol, 73 : 8902-8906, 1999; Decaussin, G., et al., Expression of BARF1 gene encoded by Epstein-Barr virus in nasopharyngeal carcinoma biopsies. Cancer Res, 60 : 5584-5588, 2000.)

NPC oncogenesis is also promoted by cellular gene alterations. Inactivation of the p16/INK4 gene by point mutations or hypermethylation is the most consistent of these alterations. (Lo, K. W., et al., Hypermethylation of the p16 gene in nasopharyngeal carcinoma. Cancer Res, 56 : 2721-2725, 1996.) Small homozygous deletions or losses of heterozygocity frequently occur in chromosome 3p suggesting that this region contains a tumor-suppressor gene which is critical for NPC-oncogenesis. (Lo, K. W., et al., High resolution allelotype of microdissected primary nasopharyngeal carcinoma. Cancer Res, 60 : 3348-3353, 2000.) In contrast with data recorded in most other human epithelial malignancies, p53 is rarely mutated in NPC. (Effert, P., et al., Alterations of the p53 gene in nasopharyngeal carcinoma. J Virol, 66 : 3768-3775, 1992.) A functional inactivation of p53 has been suspected by some authors but the mechanism of this potential inactivation remains to be elucidated. (Fries, K. L. et al., Epstein-Barr virus latent membrane protein 1 blocks p53-mediated apoptosis through the induction of the A20 gene. J Virol, 70 : 8653-8659, 1996.)

Malignant NPC cells have a short doubling time and a high metastatic potential. Nevertheless, they are prone to enter apoptosis both in situ and in vitro. In the majority of cases, the apoptotic index is high on tissue sections of NPC's. (Ham, H. J., et al., Apoptosis in nasopharyngeal carcinoma as related to histopathological characteristics and clinical stage. Histopathology, 33 : 117-122, 1998.) In addition, it has been shown that NPC cells strongly express CD95 and are highly sensitive to CD95-mediated apoptosis in vitro. (Sbih-Lammali, F., et al., Control of apoptosis in Epstein Barr virus-positive nasopharyngeal carcinoma cells: opposite effects of CD95 and CD40 stimulation. Cancer Res, 59 : 924-930, 1999.)

Vulnerability to apoptosis may in part explain why NPC's have a higher sensitivity to radiotherapy and chemotherapy than most other head and neck carcinomas. Radiotherapy is the basic therapeutic arm for treatment of the primary tumors, but in a growing number of cases it is combined with induction, concomitant or adjuvant chemotherapy. (Ali, H. et al., Chemotherapy in advanced nasopharyngeal cancer. Oncology (Huntingt), 14:1223-1230, 2000.) Short term results of induction chemotherapy are often remarkable. The rate of complete remissions is routinely of 10 to 25% following two to three courses of induction chemotherapy. (Benasso, M., et al., Induction chemotherapy followed by alternating chemo-radiotherapy in stage IV undifferentiated nasopharyngeal carcinoma. Br J Cancer, 83 : 1437-1442, 2000; Chua, D. T., et al., Patterns of failure after induction chemotherapy and radiotherapy for locoregionally advanced nasopharyngeal carcinoma: the Queen Mary Hospital experience. Int J Radiat Oncol Biol Phys, 49 : 1219-1228, 2001.) In some studies, the rate of complete lymph node regressions can reach 50%. (Frikha, M., et al., Evaluation of tumoral and lymph node response to neoadjuvant chemotherapy in undifferentiated nasopharyngeal carcinoma, Bull Cancer, 84 : 273-276, 1997.) However, these good responses are unpredictable and often of short duration. In addition, in only a few studies were good short term responses to induction chemotherapy associated with some improvement in long term survival. (Benasso, M., et al., Induction chemotherapy followed by alternating chemo-radiotherapy in stage IV undifferentiated nasopharyngeal carcinoma. Br J Cancer, 83 : 1437-1442, 2000.)

So far, despite its major role in the treatment of NPC, the effects of chemotherapy on EBV-positive NPC cells have been poorly investigated. One major reason is the extreme difficulty of growing NPC cells in vitro. For several decades the only source of experimental NPC materials were tumor lines propagated into nude mice as xenografts and only a small fraction of clinical NPC specimens could be successfully grafted. (Busson, P., et al., Establishment and characterization of three transplantable EBV-containing nasopharyngeal carcinomas. Int J Cancer, 42:599-606, 88.) More recently, there were reports of NPC cell lines propagated in vitro but many of these lines do not contain EBV, thus may not be truly representative of in vivo NPC cells. (Lin, C. T., et al., Association of Epstein-Barr virus, human papilloma virus, and cytomegalovirus with nine nasopharyngeal carcinoma cell lines. Lab Invest, 71:731-736., 1994.) Significant progress was made by derivation from a Chinese NPC xenograft called Xeno-666, of a subclone called C666-1 which can be permanently grown in vitro and which retains the EBV genome. (Cheung, S. T., et al., Nasopharyngeal carcinoma cell line (C666-1) consistently harbouring Epstein-Barr virus. Int J Cancer, 83:121-126, 1999.) We have also made progress in improving short term in vitro cultures of cells derived from other NPC xenografts, especially the C15 North African NPC xenograft.

The biological mechanisms which underlie the cytotoxic effects of anti-neoplastic drugs in NPC cells has yet to be fully elucidated. There are few reports in the literature on this topic and these have been focused mainly on the effects of cisplatin and taxol. Cisplatin was shown to induce growth arrest in the CNE1 NPC cell line at concentrations of about 1 µM. This growth arrest was accompanied by increased expression of senescence-associated β-galactosidase. (Wang, X., et al., Evidence of cisplatin-induced senescent-like growth arrest in nasopharyngeal carcinoma cells. Cancer Res, 58 : 5019-5022, 1998; Wang, X., et al., Mechanism of differential sensitivity to cisplatin in nasopharyngeal carcinoma cells. Anticancer Res, 21 : 403-408, 2001.) Taxol was shown to induce growth arrest and/or apoptosis in TWO1 and TW039 NPC cell lines. (Lou, P.J. et al., Taxol reduces cytosolic E-cadherin and beta-catenin levels in nasopharyngeal carcinoma cell line TW-039: cross-talk between the microtubule- and actin-based cytoskeletons. J Cell Biochem, 79 : 542-556, 2000; Huang, T. S., et al., Activation of MAD 2 checkprotein and persistence of cyclin B1/CDC2 activity associate with paclitaxel-induced apoptosis in human nasopharyngeal carcinoma cells. Apoptosis, 5:235-241, 2000.) Significantly, however, the foregoing studies were performed on EBV-negative cell lines which either had been derived from rare forms of EBV-negative differentiated NPC's (CNE1, TW039), or had lost the EBV-genome after a few passages *in vitro* (TWO1). (Lin, C. T., et al., Association of Epstein-Barr virus, human papilloma virus, and cytomegalovirus with nine nasopharyngeal carcinoma cell lines. Lab Invest, 71:731-736., 1994.) Thus the target cell lines of the foregoing studies cannot be regarded as truly representative of undifferentiated EBV-positive NPC's.

Testing anti-neoplastic drugs on genuine EBV-positive NPC cells *in vitro* recently was made possible by technical developments in the handling of the C666-1 and C15 NPC tumor lines. As noted above, C666 was derived from the xeno 666 transplanted NPC. C666 was subsequently subcloned in order to stabilize its association with the EBV-genome, C666-1 being one of the resulting clones. In contrast, a permanent *in vitro* cell line has yet to be derived from the C15 transplanted NPC. However the selective *in vitro* growth of malignant C15 cells can be and was obtained through the use of a PolyHema matrix. The PolyHema matrix prevented the proliferation of murine fibroblasts and allowed for the proliferation of C15 cells in the form of small floating aggregates.

### Farnesyl tranferase inhibitors (FTI's)

Farnesyl tranferase inhibitors (FTI's) are molecularly targeted drugs which have been observed to induce malignant cell apoptosis in some experimental systems. There are some indications that FTI's can increase the benefit of chemotherapy or radiotherapy without a parallel increase in undesirable side-effects. (Moasser, M. M. et al., Farnesyl transferase inhibitors cause enhanced mitotic sensitivity to taxol and epothilones. Proc Natl Acad Sci USA, 95:1369-1374, 1998; Sebti, S. M. et al., Farnesyltransferase and geranylgeranyltransferase I inhibitors and cancer therapy: lessons from mechanism and bench-to-bedside translational studies. Oncogene, 19: 6584-6593, 2000.) Although FTI's were initially designed to inhibit the farnesylation and membrane anchoring of ras proteins, it is now clear that they interfere with farnesylation of other proteins, as well, especially Rho B, rap 2, and lamin A and B. Cell treatment with FTI's has been observed to result in the accumulation of geranylated Rho B which induces apoptosis of transformed cells. (Prendergast, G. C. et al., Farnesyltransferase inhibitors: antineoplastic properties, mechanisms of action, and clinical prospects. Semin Cancer Biol, 10 : 443-452., 2000.) In other cellular models FTI's have been shown to inhibit the PI-3 kinase pathway. (Plo, I., et al., The phosphoinositide 3-kinase/Alct pathway is activated by daunorubicin in human acute myeloid leukemia cell lines. FEBS Lett, 452 : 150-154., 1999.)

### Anthracyclins

Anthracyclins such as doxorubicin classically exert their cytotoxic effect by inducing production of reactive oxygen species (ROS). ROS can induce DNA lesions, p53 activation resulting in up-regulation of p21/wafl, GADD 45, CD95 and Bax. (Kostic, C. et al., Isolation and characterization of sixteen novel p53 response genes. Oncogene, 19 : 3978-3987., 2000.) ROS also have some impact on membrane lipids and can induce production of lipid second messengers. (Bettaieb, A., et al., Daunorubicin- and mitoxantrone-triggered phosphatidylcholine hydrolysis: implication in drug-induced ceramide generation and apoptosis. Mol Pharmacol, 55: 118-125., 1999.)

Presently it is difficult to know where and how the FTI- and doxorubicin-modified pathways intersect in such a way that they induce apoptosis. It has been postulated that caspase 8 is a key-effector of TRAF1-cleavage during CD-95-mediated apoptosis. (Leo, E., et al., TRAF1 is a substrate of caspases activated during tumor necrosis factor receptor-α induced apoptosis. J Biol Chem, 55:8087-8093, 2000.) However, the cleavage of TRAF1 induced by drugs has some distinct features when compared to the cleavage induced by CD95-agonists. Among these features is a sustained high ratio of uncleaved to cleaved forms of TRAF-1 in drug-treated samples, suggesting that an increase in TRAF1 production occurs in drug-treated NPC cells in parallel with the cleavage process. Interestingly, the same high ratio of cleaved to uncleaved molecules was reported by Leo et al. for the HT1080 cell line expressing exogenous TRAF1 and treated by doxorubicin. (Id.)

TRAF1 has a very restricted tissue distribution. As in the case of EBV-transformed B-lymphocytes the strong expression of TRAF1 in NPC is likely to be related to the presence of EBV. (Mosialos, G., et al., The Epstein-Barr virus transforming protein LMP1 engages signaling proteins for the tumor necrosis factor receptor family. Cell, 80 : 389-399, 1995.) Thus cleavage of TRAF1 would be a useful marker for specifically monitoring chemo-induced apoptosis in NPC cells, for example in biopsy material.

In the cell viability assay, below, both C15 and C666-1 were observed to be remarkably sensitive to the cytotoxic effects of doxorubicin at concentrations below 1 µM. However, neither doxorubicin nor taxol induced apoptosis on its own. Interestingly, doxorubicin was included in the combination of drugs that had achieved a marked - although transient - tumor regression in the patient who was the donor of the C15 tumor line.

Doxorubicin was combined with FTI's in order to achieve chemo-induced apoptosis of NPC cells and to increase cytotoxicity. Compound A is a selective inhibitor of the farnesyl-transferase enzyme having the following structure: 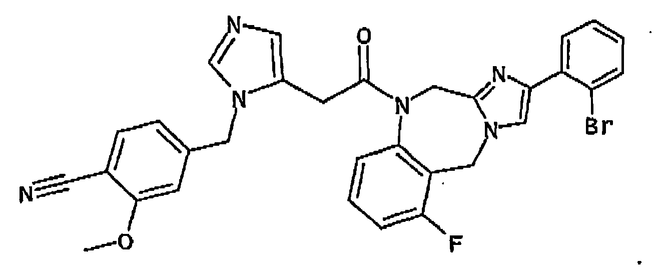 The cytotoxic effect of doxorubicin against both C15 and C666-1 cells was dramatically enhanced when it was used in combination with Compound A. The cytotoxic effect of against C15 cells was associated with massive apoptosis, associated with early cleavage of TRAF1, 48 h after starting cell exposure to the combination. The cytotoxic effect against C666-1 cells was associated with growth arrest and non-characterized cell death, apoptotic events not being observed at the tested concentrations.

It is noteworthy that in the same assay, below, both C15 cells and even more noticeably C666-1 cells were less sensitive to cis-platin than to doxorubicin. This result is consistent with a previous report of poor sensitivity of C666-1 cells to cis-platinum in a short term cytotoxicity assay. (Weinrib, L., et al., Cisplatin chemotherapy plus adenoviral p53 gene therapy in EBV-positive and -negative nasopharyngeal carcinoma. Cancer Gene Their, 8: 352-360., 2001.) These results are surprising since cis-platinum is currently regarded as the most effective agent in the chemotherapy of NPC's. (Ali, H. et al., Chemotherapy in advanced nasopharyngeal cancer. Oncology (Huntingt), 14 : 1223-1230, 2000.)

### SUMMARY OF THE INVENTION

In a first aspect the present invention features a pharmaceutical composition comprising a farnesyl transferase inhibitor, or a pharmaceutically acceptable salt thereof , and an anthracycline or a pharmaceutically acceptable salt thereof wherein said farnesyl transferase inhibitor is a compound which is disclosed in International Patent Application Publication No. WO 00/39130; i.e., a compounds according to formula I: 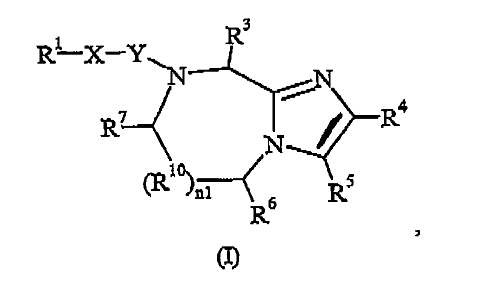 wherein
n1 is 1;
X is, independently for each occurrence, (CHR¹¹)ₙ₃(CH₂)ₙ₄Z(CH₂)ₙ₅;
Z is a bond;
Z is a bond;
n3 is, independently for each occurence, 0 or 1;
n4 and n5 each is, independently for each occurrence, 0,1,2, or 3;
Y is, independently for each occurrence, CO;
R¹ is 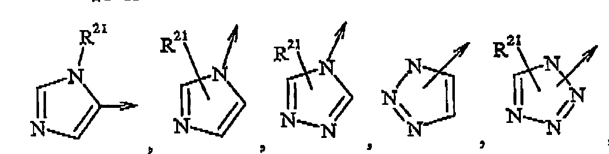 or 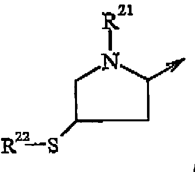
R², and R¹² each is, independently for each occurrence, H or an optionally substituted moiety selected from the group consisting of (C₁₋₆)alkyl and aryl, wherein said optionally substituted moiety is optionally substituted with one or more of R⁸ or R³⁰;
R¹¹ is independently for each occurance, H;
R³ is, independently for each occurrence, H or an optionally substituted moiety selected from the group consisting of (C₁₋₆)alkyl, (C₂₋₆)alkenyl, (C₂₋₆)alkynyl, (C₃₋₆)cycloalkyl, (C₃₋₆)cycloalkyl(C₁₋₆)alkyl, (C₅₋₇)cycloalkenyl, (C₅₋₇)cycloalkenyl(C₁₋₆)alkyl, aryl, aryl(C₁₋₆)alkyl, heterocyclyl, and heterocyclyl(C₁₋₆)alkyl, wherein said optionally substituted moiety is optionally substituted with one or more R³⁰;
R⁴ phenyz is, independently for each occurrence, an optionally substituted phenyl moiety , wherein said optionally substituted phenyl moiety is optionally substituted with one or more R³⁰, wherein each said substituent is independently selected,
R⁵ is, independently for each occurance, H;
R⁶ is, independently for each occurrence, H or an optionally substituted moiety selected from the group consisting of (C₁₋₆)alkyl, (C₂₋₆)alkenyl, (C₃₋₆)cycloalkyl, (C₃₋₆)cycloalkyl(C₁₋₆)alkyl, (C₅₋₇)cycloalkenyl, (C₅₋₇)cycloalkenyl(C₁₋₆)alkyl, aryl, aryl(C₁₋₆)alkyl, heterocyclyl, and heterocyclyl(C₁₋₆)alkyl, wherein said optionally substituted moiety is optionally substituted with one or more substituents each independently selected from the group consisting of OH, (C₁₋₆)alkyl, (C₁₋₆)alkoxy, -N(R⁸R⁹), -COOH, -CON(R⁸R⁹), and halo,
   where R⁸ and R⁹ each is, independently for each occurrence, H, (C₁₋₆)alkyl, (C₂₋₆)alkenyl, (C₂₋₆)alkynyl, aryl, or aryl(C₁₋₆)alkyl;
R⁷ is, independently for each occurrence, H, =O, =S, or an optionally substituted moiety selected from the group consisting of (C₁₋₆)alkyl, (C₂₋₆)alkyl, (C₃₋₆)cycloalkyl, (C₃₋₆)cycloalkyl(C₁₋₆)alkyl, (C₅₋₇)cycloalkenyl, (C₅₋₇)cycloalkenyl(C₁₋₆)alkyl, aryl, aryl(C₁₋₆)alkyl, heterocyclyl, and heterocyclyl(C₁₋₆)alkyl, wherein said optionally substituted moiety is optionally substituted with one or more substituents each independently selected from the group consisting of OH, (C₁₋₆)alkyl, (C₁₋₆)alkoxy, -N(R⁸R⁹), -COOH, -CON(R⁸R⁹), and halo;
R¹⁰ is C;
R²¹ is, independently for each occurrence, H or an optionally substituted moiety selected from the group consisting of (C₁₋₆)alkyl and aryl(C₁₋₆)alkyl, wherein said optionally substituted moiety is optionally substituted with one or more substituents each independently selected from the group consisting of R⁸ and R³⁰; R²² is H, (C₁₋₆)alkylthio, (C₃₋₆)cycloalkylthio, R⁸-CO-, or a substituent according to the formula 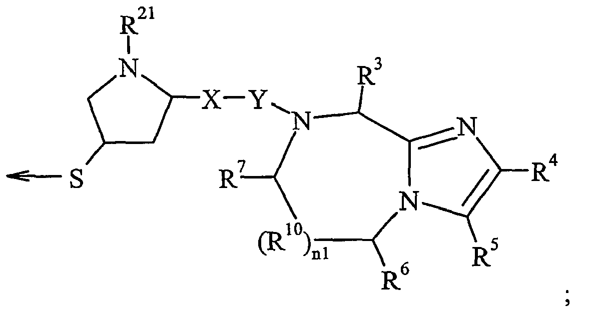
R²⁴ and R²⁵ each is, independently for each occurrence, H, (C₁₋₆)alkyl, or aryl(C₁₋₆)alkyl;
R³⁰ is, independently for each occurrence, (C₁₋₆)alkyl, -O-R⁸, -S(O)ₙ₆R⁸, - S(O)ₙ₇N(R⁸R⁹), -N(R⁸R⁹), -CN, -NO₂, -CO₂R⁸, -CON(R⁸R⁹), -NCO-R⁸, or halogen; n6 and n7 each is, independently for each occurrence, 0, 1, or 2;
   wherein said heterocyclyl is azepinyl, benzimidazolyl, benzisoxazolyl, benzofurazanyl, benzopyranyl, benzothiopyranyl, benzofuryl, benzothiazolyl, benzothienyl, benzoxazolyl, chromanyl, cinnolinyl, dihydrobenzofuryl, dihydrobenzothienyl, dihydrobenzothiopyranyl, dihydrobenzothio-pyranyl sulfone, furyl, imidazolidinyl, imidazolinyl, imidazolyl, indolinyl, indolyl; isochromanyl, isoindolinyl, isoquinolinyl, isothiazolidinyl, isothiazolyl, isothiazolidinyl, morpholinyl, naphthyridinyl, oxadiazolyl, 2-oxoazepinyl, 2-oxopiperazinyl, 2-oxopiperidinyl, 2-oxopyrrolidinyl, piperidyl, piperazinyl, pyridyl, pyridyl N-oxide, quinoxalinyl, tetrahydrofuryl, tetrahydroisoquinolinyl, tetrahydro-quinolinyl, thiamorpholinyl, thiamorpholinyl sulfoxide, thiazolyl, thiazolinyl, thienofuryl, thienothienyl, or thienyl; and
   wherein said aryl is phenyl or naphthyl;
   provided that:
R⁶ is H, and R¹⁰ and R⁷ are taken together to form 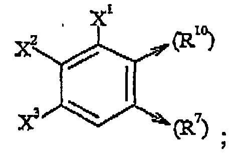 or
R⁷ is =O, -H, or =S, and R¹⁰ and R⁶ and taken together to form 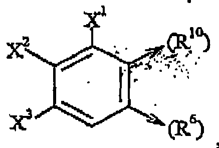 wherein X¹, X², and X³ each is, independently, H, halogen, -NO₂, -NCO-R⁸, -CO₂R⁸, -CN, or -CON(R⁸R⁹);
or a pharmaceutically acceptable salt thereof.

According to a second preferred embodiment of the first aspect of the invention said farnesyl transferase inhibitor is a compound according to formula I,
wherein:
R¹ is 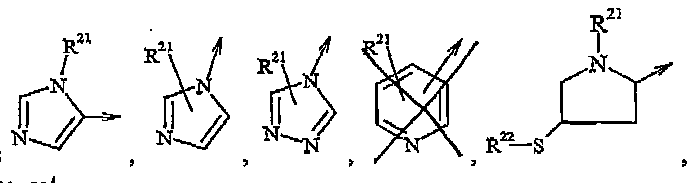 or ; and
X is CH(R¹¹)ₙ₃(CH₂)ₙ₄ ;
or a pharmaceutically acceptable salt thereof.

According to a third more preferred embodiment of said second preferred embodiment of the first aspect of the invention said farnesyl transferase inhibitor is a compound according to formule I, wherein:
R¹ is 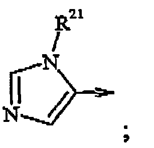
R⁶ is H;
n1 is 1;
R⁷ and R¹⁰ are taken together to form 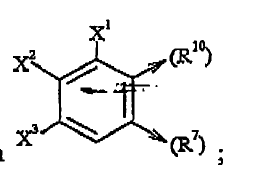
n3 is 1 and R¹¹ is H;
Z is a bond,
n5 is 0; and
Y is CO,
or a pharmaceutically acceptable salt thereof.

According to a fifth more preferred embodiment of said second preferred embodiment of the first aspect of the invention said farnesyl transferase inhibitor is a compound according to formula I, wherein:
R¹ is 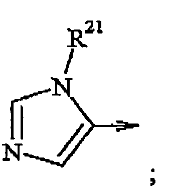
R⁷ is H or =O;
n1 is 1;
R⁶ and R¹⁰ are taken together to form 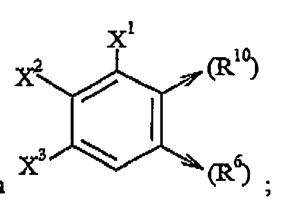
n3 is 1 and R¹¹ is H;
n5 is 0;
Y is CO ; and
Z is a bond;
or a pharmaceutically acceptable salt thereof.

According to a fourth preferred embodiment of the first aspect of the invention said farnesyl transferase inhibitor is a compound according to formula I, wherein said compound is:
1-(2-(1-(4-cyanophenylmethyl)imidazol-4-yl)-1-oxoethyl)-1,2-dihydro-4-(2-methoxyphenyl)-imdazo[1,2-c][1,4]benzodiazepine ;
9-bromo-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
9-Chloro-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
10-Bromo-1-(2-(1-(4-cyanophenyklmethyl)imidazol-4-yl)-1-oxoethyl)-1,2-dihydro-4-(2-methoxyphenyl)-imidazo[1,2-c][1,4]benzodiazepine;
1-(2-(1-(4-cyanophenylmethyl)imidazol-4-yl)-1-oxoethyl)-1,2-dihydro-8-fluoro-4-(2-methoxyphenyl)-imidazo[1,2-c][1,4]benzodiazepine; or
or a pharmaceutically acceptable salt thereof.

According to a more preferred embodiment of said fourth preferred embodiment of the first aspect of the invention said farnesyl transferase inhibitor is a compound according to formula I, wherein said compound is:
1-(2-(1-(4-cyanophenylmethyl)imidazol-4-yl)-1-oxoethyl)-1,2-dihydro-4-(2-methoxyphenyl)-imidazo[1,2-c][1,4]benzodiazepine ;
9-bromo-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
9-Chloro-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
10-Bromo-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
1-(2-(1-(4-cyanophenylmethyl)imidazol-4-yl)-1-oxoethyl)-1,2-dihydro-8-fluoro-4-(2-methoxyphenyl)-imidzo[1,2-c][1,4]benzodiazepine;

According to a sixth preferred embodiment of the first aspect of the invention said farnesyl transferase inhibitor is a compound according to formula I, wherein said compound is:
5-(2-(1-(4-cyanophenylmethyl)-imidazol-5-yl)-1-oxo-ethyl)-5,6-dihydro-2-phenyl-1H-imidazo[1,2-a][1,4]benzodiazepine;
or a pharmaceutically acceptable salt thereof.

According to a seventh preferred embodiment of the first aspect of the invention said farnesyl transferase inhibitor is a compound according to formula I, wherein said compound is:
1,2-dihydro-1-(2-(imidazol-1-yl)-1-oxoethyl)-4-(2-methoxyphenyl) imidazo[1,2a][1,4]benzodiazopine;
or a pharmaceutically acceptable salt thereof.

According to an eighth preferred embodiment of the first aspect of the invention said farnesyl transferase inhibitor is a compound according to formula I,
wherein said compound is: 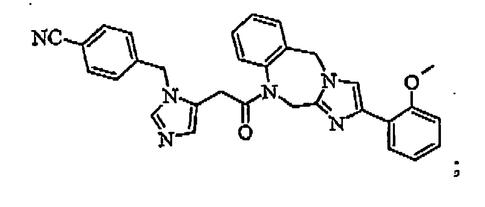 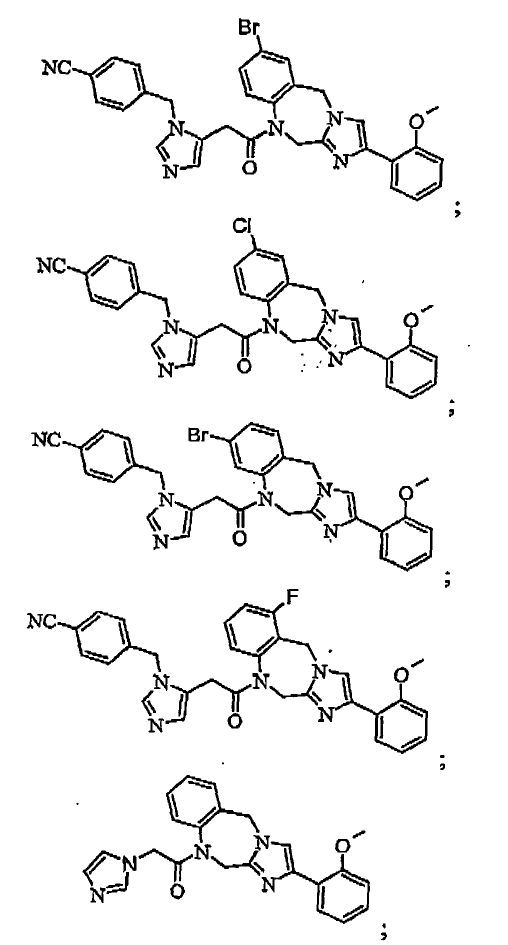 or 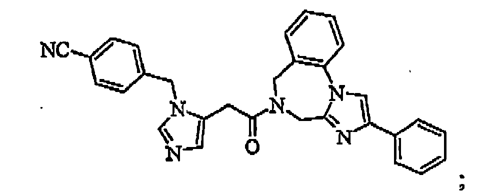 or a pharmaceutically acceptable salt thereof.

According to a ninth preferred embodiment of the first aspect of the invention said farnesyl transferase inhibitor is a compound according to formula I, wherein said compound is: 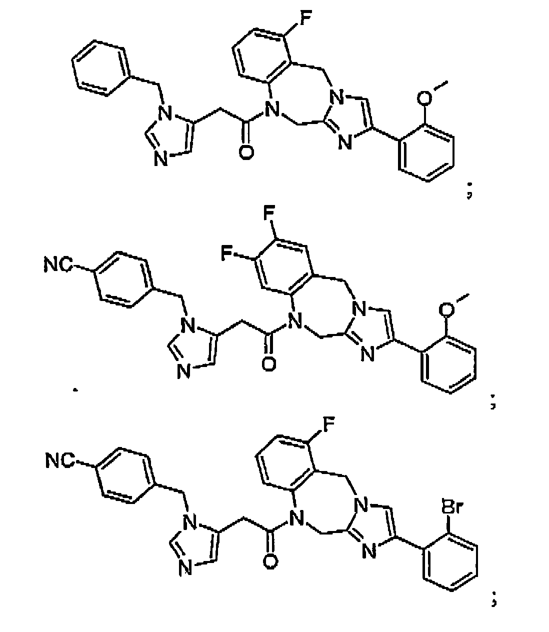 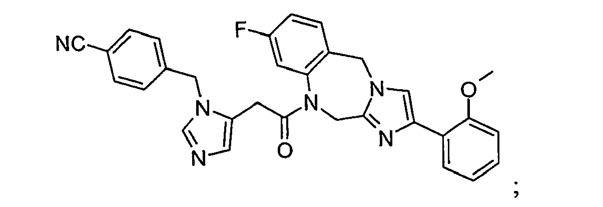 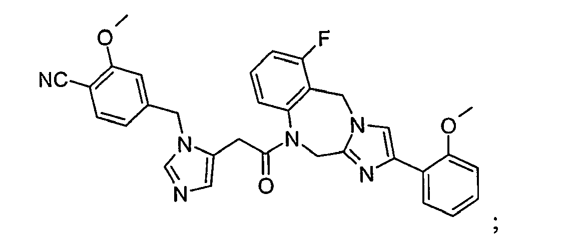 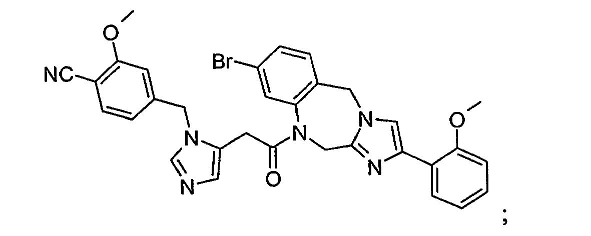 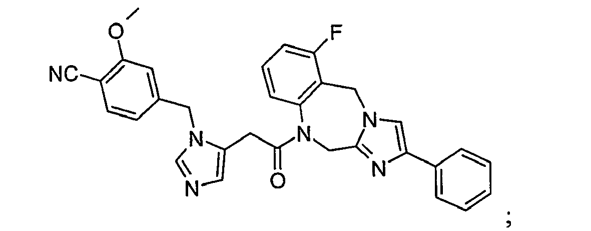 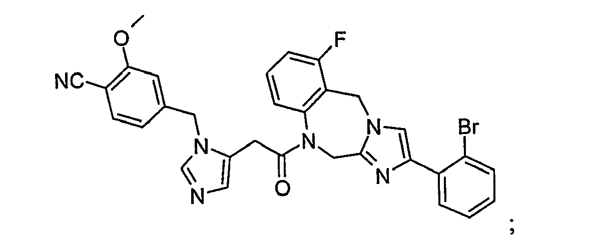 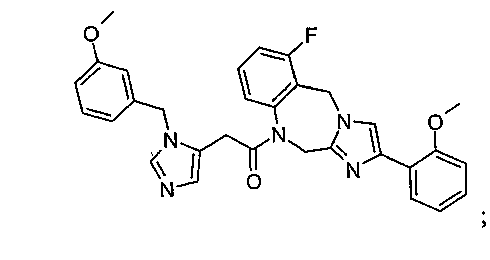 or 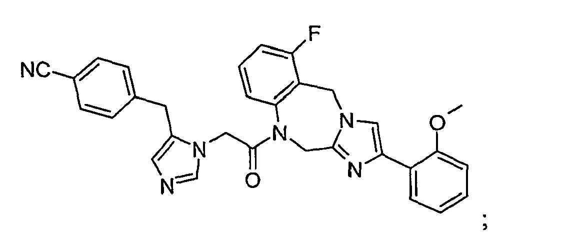 or a pharmaceutically acceptable salt thereof.

According to a first more preferred embodiment of said ninth preferred embodiment of the first aspect of the invention said farnesyl transferase inhibitor is a compound according to formula I, wherein said compound is: 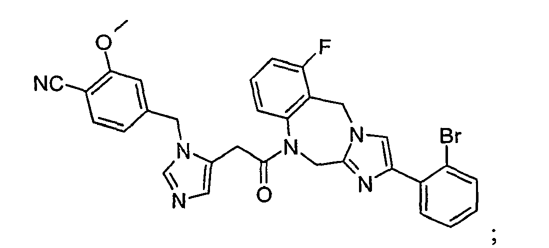 or a pharmaceutically acceptable salt thereof.

A second more preferred embodiment of said ninth preferred embodiment comprises said first more preferred embodiment of said eighth preferred embodiment wherein said anthracyclin is doxorubicin, daunorubicin, epirubicin, idarubicin, or amrubicin, or a pharmaceutically acceptable salt thereof.

A third more preferred embodiment of said ninth preferred embodiment comprises said second more preferred embodiment of said eighth preferred embodiment wherein said anthracyclin is doxorubicin, or a pharmaceutically acceptable salt thereof.

In a preferred embodiment of the first aspect of the invention, and of each of said first through ninth preferred embodiments of said first aspect of the invention, and of each of said more preferred embodiments thereof, termed the tenth preferred embodiment of said first aspect of the invention, said anthracycline is doxorubicin, daunorubicin, epirubicin, idarubicin, or amrubicin, or a prodrug thereof, or a pharmaceutically acceptable salt of said anthracyclin or of said anthracyclin prodrug.

In a more preferred embodiment of said tenth preferred embodiment, said anthracyclin is doxorubicin, or a pharmaceutically acceptable salt thereof.

In a second aspect the present invention features a pharmaceutical composition according to said first aspect of the invention, or according to any one of said preferred embodiments of said first aspect, or according to any one of said more preferred embodiments of said first aspect, and a pharmaceutically acceptable carrier, vehicle or diluent.

In a third aspect the present invention features a method of decreasing the rate of proliferation of nasopharyngeal carcinoma cells, said method comprising contacting said nasopharyngeal cells with a farnesyl transferase inhibiting compound in combination with an anthracycline compound, separately or simultaneously, wherein each of said farnesyl transferase inhibiting compound and said anthracycline compound is selected from the farnesyl transferase inhibiting compounds the anthracycline compounds disclosed in the pharmaceutical compositions according to said first aspect of the invention, or according to any one of said preferred embodiments of said first aspect, or according to any one of said more preferred embodiments of said first aspect.

In a first preferred embodiment of said third aspect the present invention features a method of decreasing the rate of proliferation of nasopharyngeal carcinoma cells, said method comprising contacting said nasopharyngeal cells with a pharmaceutical composition according to said first aspect of the invention, or according to any one of said preferred embodiments of said first aspect, or according to any one of said more preferred embodiments of said first aspect

In a second preferred embodiment of said third aspect the present invention features a method of treating nasopharyngeal carcinoma in a patient, said method comprising administering to said patient a pharmaceutical composition according to said first aspect of the invention, or according to any one of said preferred embodiments of said first aspect, or according to any one of said more preferred embodiments of said first aspect.

In a third preferred embodiment of said third aspect the present invention features a method of treating nasopharyngeal carcinoma in a patient, said method comprising administering to said patient effective amounts of one or more farnesyl transferase inhibitor in combination with one or more anthracycline, wherein said effective amounts of said farnesyl transferase inhibitor or inhibitors and of said anthracyclin or anthracyclines are effective in combination to treat said nasopharyngeal carcinoma.

In a fourth preferred embodiment of said third aspect, the invention features a method according to any one of said first, second or third preferred embodiments of said fourth preferred embodiments wherein said patient is a mammal.

In a fifth preferred embodiment of said third aspect, the invention features a method according to any one of said first, second, third or fourth preferred embodiments of said third aspect wherein said patient is a human being.

In a sixth preferred embodiment of said third aspect, the invention features a method according to any one of said first, second, third, fourth or fifth preferred embodiments of said third aspect wherein a farnesyl transferase inhibitor and an anthracycline are administered substantially simultaneously.

In a fourth aspect the invention features a pharmaceutical kit comprising one or more of a farnesyl transferase inhibitor or pharmaceutically acceptable salt thereof and one or more of an anthracycline or pharmaceutically acceptable salt thereof and instructions for use for the treatment of nasopharyngeal carcinoma.

In a first preferred embodiment of said fourth aspect of the invention features a kit comprising: a) a first unit dosage form comprising a farnesyl transferase inhibitor, a prodrug thereof or a pharmaceutically acceptable salt of said farnesyl transferase inhibitor or of said farnesyl transferase inhibitor prodrug and a pharmaceutically acceptable carrier, vehicle or diluent; b) a second unit dosage form comprising an anthracycline, a prodrug thereof or a pharmaceutically acceptable salt of said anthracycline or of said anthracycline prodrug and a pharmaceutically acceptable carrier, vehicle or diluent; and c) a container.

In a more preferred embodiment of said first preferred embodiment of said fourth aspect of the invention is featured a kit wherein said farnesyl transferase inhibitor comprises a compound according to formula I or a pharmaceutically acceptable salt thereof, and said anthracycline comprises doxorubicin, daunorubicin, epirubicin, idarubicin, or amrubicin or a pharmaceutically acceptable salt thereof.

In a still more preferred embodiment of said first preferred embodiment of said fourth aspect of the invention is featured a kit wherein said farnesyl transferase inhibitor comprises a compound according to the formula: 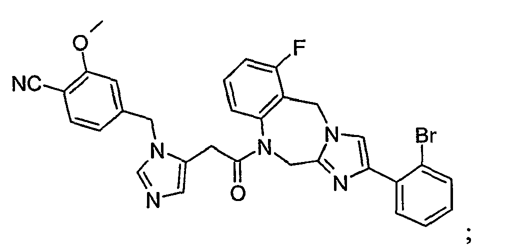 or a pharmaceutically acceptable salt thereof, and said anthracycline comprises doxorubicin or a pharmaceutically acceptable salt thereof.

The disclosure of International Patent Application Publication No. WO 00/39130 teaches one or more methods of synthesizing compounds according to formula I.

Particularly preferred are the farnesyl transferase inhibitor compounds described in the following international patent applications and publications: WO00/39130, WO98/00409, WO99/65922, WO99/65898, WO99/64401, and PCT/US01/23959.

According to yet another preferred aspect, the present invention relates to a drug combination comprising at least one anthracycline compound and at least one farnesyl transferase inhibitor compound, wherein said anthracycline compound is described in one or more of the following United States patents : 6,437,105, 6,433,150, 6,403,563, 6,284,738, 6,284,737, 6,245,358, 6,194,422, 6,187,758, 6,184,374, 6,184,365, 6,160,102, 6,107,285, 6,103,700, 6,087,340, 6,080,396, 5,977,082, 5,965,407, 5,958,889, 5,948,896, 5,945,518, 5,942,605, 5,843,903, 5,801,257, 5,789,386, 5,776,458, 5,744,454, 5,719,130, 5,710,135, 5,593,970, 5,587,495, 5,532,218, 5,294,701, 5,260,425, 5,242,901, 5,220,001, 5,212,291, 5,196,522, 5,162,512, 5,124,318, 5,124,317, 5,122,368, 5,091,373, 5,091,372, 5,045,534, 5,004,606, 5,003,055, 4,997,922, 4,965,352, 4,959,460, 4,952,566, 4,950,738, 4,948,880, 4,946,831, 4,918,173, 4,918,172, 4,914,191, 4,897,470, 4,870,058, 4,863,739, 4,855,414, 4,840,938, 4,839,346, 4,833,241, 4,795,808, 4,772,688, 4,734,493, 4,713,371, 4,710,564, 4,697,005, 4,675,311, 4,663,445, 4,642,335, 4,632,922, 4,612,371, 4,591,636, 4,564,674, 4,563,444, 4,562,177, 4,550,159, 4,537,882, 4,534,971, 4,526,960, 4,522,815, 4,474,945, 4,472,571, 4,465,671, 4,439,603, 4,438,105, 4,424,342, 4,419,348, 4,411,834, 4,409,391, 4,405,713, 4,405,522, 4,401,812, 4,393,052, 4,387,218, 4,385,122, 4,383,037, 4,373,094, 4,366,149, 4,360,664, 4,355,026, 4,351,937, 4,337,312, 4,327,029, 4,325,946, 4,322,412, 4,309,503, 4,303,785, 4,293,546, 4,267,312, 4,264,510, 4,263,428, 4,259,476, 4,247,545, 4,245,045, 4,244,880, 4,215,062, 4,209,588, 4,207,313, 4,192,915, 4,166,848, 4,138,480, 4,134,903, 4,133,877, 4,127,714, 4,067,969, and 3,963,760.

According to yet another preferred aspect, the present invention relates to a drug combination comprising at least one anthracycline compound and at least one farnesyl transferase inhibitor compound, wherein said anthracycline compound is described in one or more of the following United States patent publications: 20020137694, 20020077303, and 20010053845.

According to still yet another preferred aspect, the present invention relates to a drug combination comprising at least one anthracycline compound and at least one farnesyl transferase inhibitor compound, wherein said anthracycline compound is described in one or more of the following international patent applications and publications, : WO0187814, WO0164197, WO0076525, WO0066093, WO0056267, W00044762, WO0027404, WO0026223, WO0015203, WO9966918, WO9958543, WO9957126, WO9952921, WO9948503, W09945015, WO9935153, W09931140, WO9929708, WO9908687, WO9846598, WO9840104, WO9839337, WO9802446, WO9749433, WO9749390, WO9740057, WO9733897, WO9729191, WO9719954, WO9712895, WO9700880, WO9639121, WO9629335, WO9607665, WO9524412, WO9516695, WO9516693, WO9509173, W09426311, W09420114, WO9405259, WO9313804, WO9305774, WO9216629, W09210212, WO9207866, WO9119725, WO9105546, WO9010639, WO9004601, WO9001490, WO9000173, WO8911532, WO8809823, WO8809166, WO8703481, W08203769 and WO8002112.

According to a more preferred aspect, the present invention relates to drug combinations comprising at least one farnesyl transferase inhibitor compound described in International Patent Publication No. WO 00/39130 with an anthracycline. Among the anthracyclines, doxorubicin, daunorubicin, epirubicin, idarubicin, and amrubicin are preferred, and doxorubicin is most preferred.

In a particularly preferred embodiment the invention features a drug combination comprising Compound A and doxorubicin.

As a result of their mechanism of action, the farnesyl transferase inhibitor compounds, especially the compounds mentioned above, in general have a cytostatic-type activity.

In employing the drug combinations according to the invention comprising at least one farnesyl transferase inhibitor and at least one anthracycline a goal is to obtain an increased anti-cancer activity, such as, for example, a prolonged stabilization of the size of the tumor, or a tumor regression.

According to the present invention, a composition is active if after its administration, it inhibits, retards or prevents the proliferation of tumor cells. Thus an advantageous characteristic of a pharmaceutical composition comprising a drug combination according to the invention can be an increase in the delay of tumor growth.

The drug combinations according to the present invention can advantageously prolong or maintain the anticancer activity of either the anthracycline compound or the farnesyl transferase compound, in comparison with the activities obtained with each of the anthracycline compound or the farnesyl transferase compound considered in isolation.

A further advantage of the combinations according to the present invention relates to toxicity. Specifically, the therapeutic synergy of the combinations allows for lower dosages of one or both of the components relative to the dosages that would be required to achieve the same level of therapeutic activity if the components were administered individually.

The term "prodrug" refers to a pharmaceutically acceptable metabolic precursor of a drug of interest; i.e., a compound or composition which is converted to an active form of a desired drug in the body.

Certain abbreviations are used herein, as follows:
- EBERs: EBV-encoded RNAs
- EBNA1: Epstein-Barr nuclear antigen 1
- EBV: Epstein-Barr virus
- Ftase: farnesyl-transferase
- GGTase: geranylgeranyl-transferase
- FTI: farnesyltransferase inhibitors
- LMP1: latent membrane protein 1
- NPC: nasopharyngeal carcinoma
- TRAF: TNF-receptor associated factor

The anthracycline compound and the farnesyl transferase inhibiting compound which form the drug combination of the invention can be administered simultaneously, separately or sequenced over time, it being possible to adapt this frequency so as to obtain the maximum efficacy of the combination and, for each administration, to have a variable duration ranging from a rapid total administration to a continuous perfusion. The compounds which form the combination can be administered at different rates, can be administered independently according to schemes chosen from the continuous, intermittent, repeated, alternated or sequential schemes, and can be repeated a number of times per day.

Advantageously, the farnesyl transferase inhibitor having a cytostatic activity can be administered according to a continuous scheme. More advantageously, this scheme allows the plasma levels to be maintained higher than or equal to the concentration necessary to inhibit 50% of the growth of the cells (IC₅₀), e.g., as determined in the in-vitro cell viability assay described herein. Advantageously, the anthracycline can be administered according to a scheme dependent on the type of tumor model; preferably according to an intermittent scheme.

It follows from the foregoing that the drug combinations of the present invention are not limited to those which are obtained by physical association of the constituents, but also to those which allow a separate administration which can be simultaneous or spread out over time. Thus the constituents can be administered independently according to distinct methods and routes including, without limitation, the oral, intraperitoneal, parenteral, intravenous, topical, rectal, vaginal, and respiratory mucosa routes.

Advantageously, the farnesyl transferase inhibitor and anthracycline constituents of the combinations according to the invention are administered orally; most preferably, the farnesyl transferase inhibitor and anthracycline constituents of the drug combinations according to the invention are bioavailable by the oral route.

Also advantageously, the farnesyl transferase inhibitor and anthracycline constituents of the drug combinations according to the invention may be administered intravenously.

Also advantageously, the farnesyl transferase inhibitor constituents may be administered orally and the anthracycline constituents may be administered intravenously, or vice versa.

The products for intravenous injection are generally pharmaceutically acceptable sterile solutions or suspensions which optionally can be prepared extemporaneously at the time of use. For the preparation of non-aqueous solutions or suspensions, natural vegetable oils can be used such as olive oil, sesame oil or paraffin oil or injectable organic esters such as ethyl oleate. The sterile aqueous solutions can be formed of a solution of one or both of the anthracycline compound and the farnesyl transferase inhibiting compound in water. The aqueous solutions are suitable for intravenous administration inasmuch as the pH is suitably adjusted and the isotonicity is produced, for example, by a sufficient quantity of sodium chloride or of glucose. Sterilization can be carried out by heating or by any other means which does not adversely affect the composition. The drug combinations can also be present in the form of liposomes or in combination form with supports such as cyclodextrins or polyethylene glycol's.

As solid compositions for oral administration, compressed tablets, pills, powders (gelatin capsules, cachets) or granules can be used. In these compositions, one or both of the anthracycline compound and the farnesyl transferase inhibiting compound is mixed with one or more inert diluents, such as starch, cellulose, sucrose, lactose or silica, under a current of argon. These compositions can likewise comprise substances other than the diluents, for example one or more lubricants such as magnesium stearate or talc, a colorant, a coating (coated tablets) or a lacquer.

As liquid compositions for oral administration, it is possible to use pharmaceutically acceptable solutions, suspensions, emulsions, syrups and elixirs comprising inert diluents such as water, ethanol, glycerol, vegetable oils or paraffin oil. These compositions can comprise substances other than the diluents, for example wetting, sweetening, thickening, flavoring or stabilising products.

The compositions for rectal administration are suppositories or rectal capsules which contain, apart from one or both of the anthracycline compound and the farnesyl transferase inhibiting compound, excipients such as cocoa butter, semi-synthetic glycerides or polyethylene glycols.

The compositions for topical administration can be, for example, creams, lotions, eye lotions, mouthwashes nasal drops or aerosols.

Generally speaking, the physician will determine the appropriate dosage of each of the anthracycline compound and the farnesyl transferase inhibiting compound as a function of the age, weight and all the other factors individual to the subject to be treated. Generally the doses depend on the effect sought, the duration of the treatment and the route of administration used. Doses generally range, as far as the farnesyl transferase inhibitor is concerned, from 10 mg to 2000 mg per day by the oral route for an adult with unit doses ranging from 50 mg to 1000 mg of active substance; and as far as the anthracyclines are concerned: from 10 mg to 1000 mg of active substance per day by the intravenous route for an adult. The treatment can be repeated a number of times per day or per week as determined appropriate by the physician. Preferably the treatment is continued until a stabilisation, a partial remission, a total remission or a recovery is achieved.

In the combinations according to the invention for which the administration of the constituents can be simultaneous, separated or spread out over time, it is particularly advantageous that the quantity of the farnesyl transferase inhibitor compound is from 10 to 90% by weight of the combination, it being possible for this content to vary as a function of the nature of the associated substance, the efficacy sought and the nature of the cancer cells to be treated.

The drug combinations according to the invention can be utilized for the treatment of diseases connected with malignant or benign cell proliferations of the cells of various tissues and/or organs, comprising the muscle, bone or connective tissues, the skin, the brain, the lungs, the sex organs, the lymphatic or renal systems, the mammary or blood cells, the liver, the digestive apparatus, the colon, the pancreas and the thyroid or adrenal glands, and including the following pathologies: psoriasis, restenosis, different types of sarcomas such as Kaposi's sarcoma, cancers of the head and of the neck, the pancreas, the colon, the lung, the ovary, the breast, the brain, the prostate, the liver, the stomach, the bladder, the kidney, the prostate or the testicles, Wilm's tumor, teratocarcinomas, cholangiocarcinoma, choriocarcinoma, melanomas, cerebral tumors such as neuroblastoma, gliomas, multiple myelomas, leukemias and lymphomas such as chronic lymphocytic leukemias, acute or chronic granulocytic lymphomas, and Hodgkin's disease.

The combinations according to the invention can be particularly useful for the treatment of cancers such as cancers of the pancreas, the colon, the lung, the ovary, the breast, the brain, the prostate, the liver, the stomach, the bladder or the testicles, and of the head and neck, and more advantageously cancer of the head and neck. In a particularly preferred embodiment of the invention a combination according to the invention is used for the treatment of nasopharyngeal carcinoma.

In particular, the drug combinations of the invention have the advantage of being able to employ the anthracycline compound and/or the farnesyl transferase inhibiting compound in doses which are lower than those in which either compound is used alone.

Thus the present invention relates to the use of combinations comprising at least one farnesyl transferase inhibitor and at least one anthracycline for the preparation of medicaments useful for the treatment of the above mentioned pathologies; advantageously cancers, most advantageously nasopharyngeal carcinoma. Further the present invention relates to the use of drug combinations comprising at least one farnesyl transferase inhibitor and at least one anthracycline for the preparation of medicaments for administration which is simultaneous, separate or sequenced over time.

The farnesyl transferase inhibitor compounds referenced herein can be prepared by means commonly known to those skilled in the art, as evidenced by the patents already specifically incorporated by reference herein. Likewise the anthracycline compounds can be prepared by means well-known in the art.

The present invention is further illustrated by the following examples which are designed to teach those of ordinary skill in the art how to practice the invention.

### BRIEF DESCRIPTION OF THE DRAWINGS

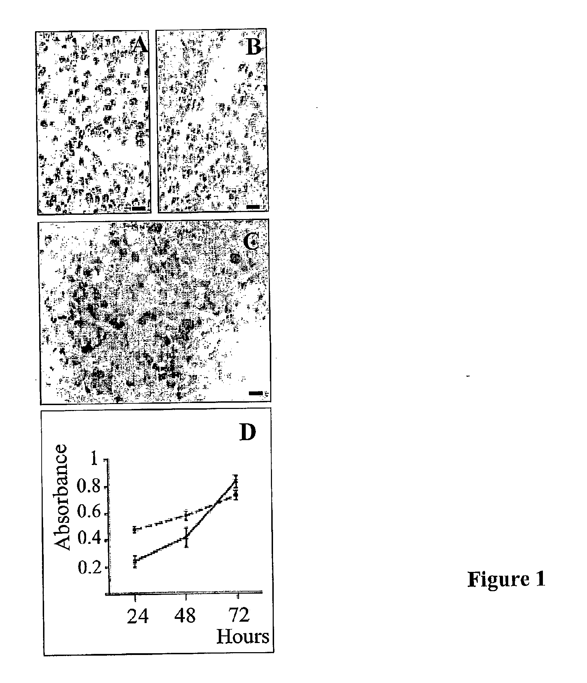

Figure 1. Detection of the EBERs in NPC tumor lines and assessment of NPC cell proliferation *in vitro.* A and B: *in situ* hybridization of the EBERs on tissue sections of xenografted tumors formed by C15 (A) and C666-1 (B) NPC cells. (scale bar: 20 µM). C: Ki 67 immunostaining of C15 cells grown *in vitro* for 72 h on PolyHema matrix; cell aggregates (average size 150 µM) were cytospined and fixed in acetone (10 min/4°C) prior to immunostaining (scale bar : 10 µm). D: Proliferation of C15 (solid line) and C666-1 (dotted line) cells *in vitro* demonstrated by sequential WST-1 assays. Cells were seeded in 96 well plates at 10⁵/well on plastic coated with PolyHema (C15) and 35 X 10³/well without plastic coating (C666-1). The WST-1 reaction was performed at 24, 48 and 72 hours to assess the evolution of cell viability reflected by absorbance at 490 nm. Data are the means (± standard deviation) of quadruplicates. Similar results were obtained in three separate experiments.

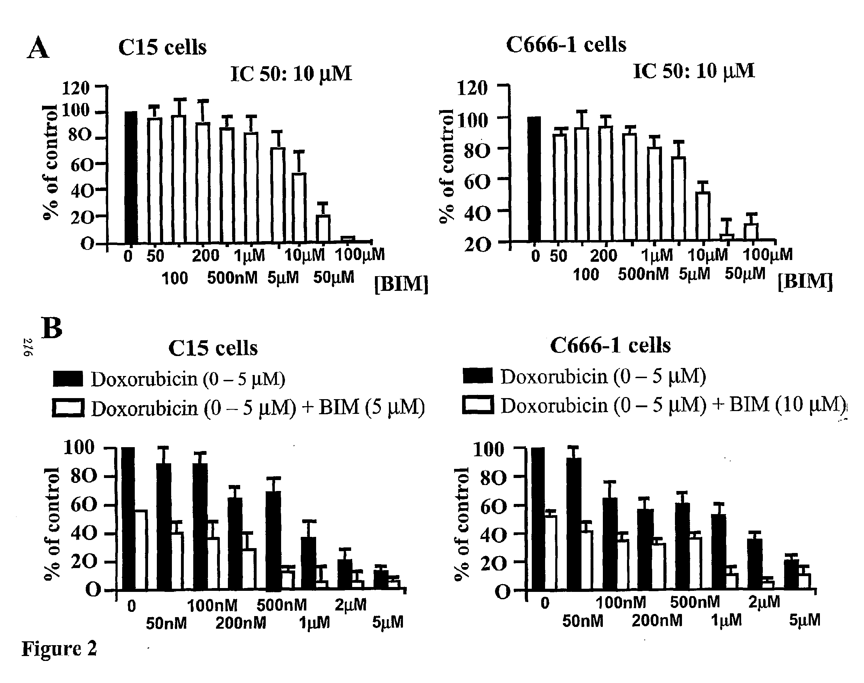

Figure 2. Effect of Compound A, alone or in combination with doxorubicin, on NPC cell viability. C15 and C666-1 cells were grown in 96 well plates, in the presence of various concentrations of pharmacological agents, prior to assessment of cell viability using the WST-1 assay. The observed results are reported as means (± standard deviation) of quadruplicates and are representative of three similar experiments. A: treament of NPC cells with various concentrations of Compound A alone. B: treament of NPC cells with various concentrations of doxorubicin with or without Compound A, 5 µM (C15) or 10 µM (C666-1).

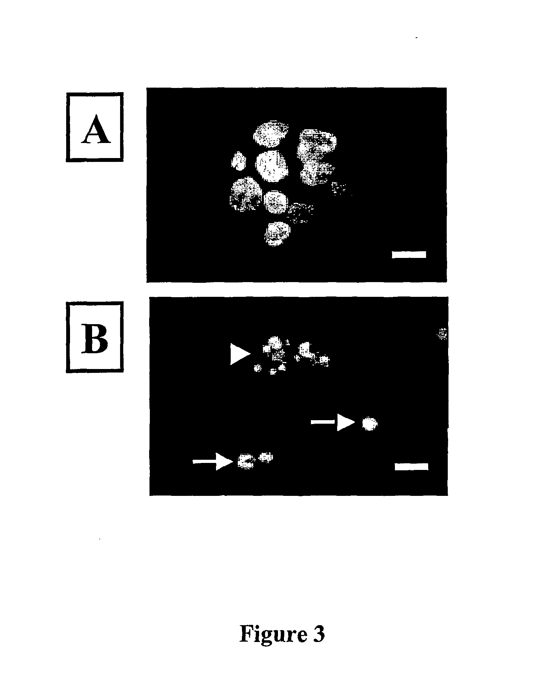

Figure 3. Induction of nuclear fragmentation in C15 cells treated by doxorubicin combined to Compound A. Nuclei were visualized by Hoechst 33342 staining (scale bar : 10 µm). A: non-treated cells. B: cells treated for 48 hrs with doxorubicin (1 µM) combined to Compound A (5 µM); most nuclei shrank (arrows) and exhibited chromatin condensation and fragmentation (arrowheads).

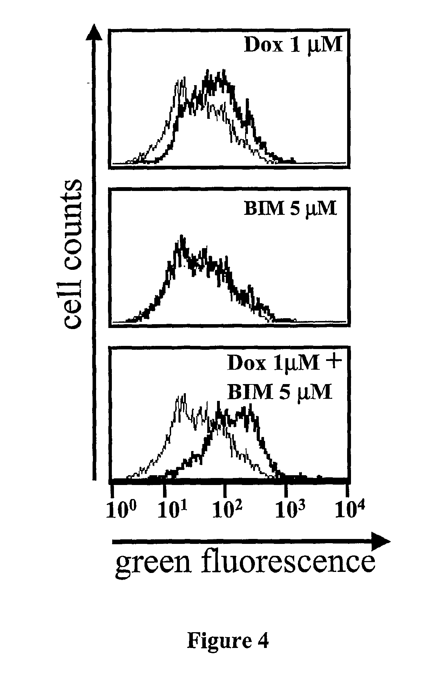

Figure 4. Induction of caspase activation in C15 cells treated by doxorubicin combined to Compound A. C15 cells were treated with doxorubicin (1 µM), Compound A (5µM) or a combination of both molecules for 48 h. Measurement of caspase activation was based on selective intra-cellular retention of a fluorescent caspase substrate inhibitor (VAD-fluoromethyl ketone labelled with carboxyfluorescein). Retention of the fluorescent substrate was assessed by flow cytometry in non-treated (grey line) or treated (black line) C15 cells.

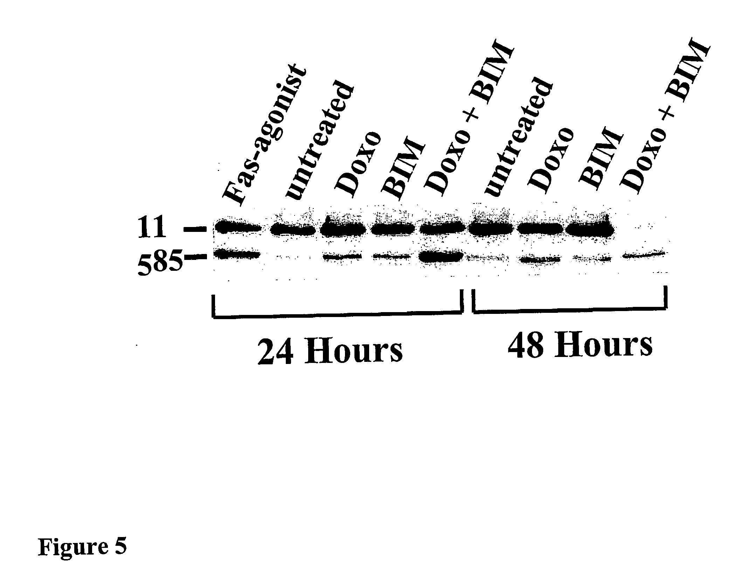

Figure 5. Induction of PARP-cleavage in C15 cells treated by doxorubicin combined to Compound A. C15 cells were treated with doxorubicin (1 µM), Compound A (5µM) or a combination of both for the indicated periods. Corresponding cell lysates (50 µg protein per lane) were separated by 7.5% SDS-PAGE and analyzed by Western Blot with an anti-PARP monoclonal antibody. The cleaved product of PARP was at 85 kD, as shown in the positive-control extract derived from C15 cells treated with the CD95 agonist-antibody 7C11.

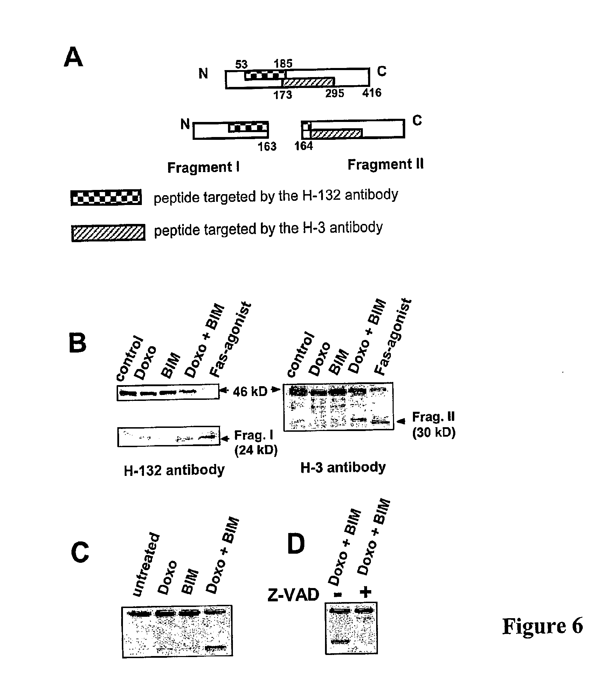

Figure 6. Induction of TRAF1-cleavage in C15 cells treated with doxorubicin combined to Compound A. A: Diagram of Fas-mediated TRAF-1-cleavage as reported by Leo et al. (2001). The locations of target peptides used for production of the H-3 monoclonal and H-132 polyclonal antibody are indicated by hatched boxes. The peptide targeted by H-3 was entirely contained in fragment II whereas the peptide targeted by H132 was mainly co-linear with fragment I. B: C15 cells were treated for 24h with doxorubicin (1 µM), Compound A (5µM) or a combination of both. Control cells were treated with the Fas-agonist antibody 7C11. Cell lysates (50 µg protein per lane) were separated on a 8-16% SDS-PAGE linear gradient prior to Western Blot analysis with anti-TRAF-1 antibodies. Both antibodies detected a main band at 46 kd Additional bands corresponding to fragments of smaller sizes were visible, mainly in samples treated with 7C11 or the combination of doxorubicin and Compound A. In the extracts of cells treated with 7C11, these smaller fragments had apparent molecular weights of 29 kD (H-3) and 24 kD (H-132) which were compatible with the sizes of TRAF1 fragments reported by Leo et al. (2001). Similar results were obtained in three similar experiments.

### DETAILED DESCRIPTION OF THE INVENTION

### Materials

Compound A and BIM-46068 were provided by Biomeasure, Incorporated (Milford, MA). FTI-277 and GGTI-286 were provided by Expansia (Aramon, France). Taxol, cis-platin, doxorubicin and 5-fluorouracyl were purchased from Sigma (Saint-Quentin Fallavier, France). C15 is an undifferentiated NPC tumor line propagated by sub-cutaneous passage into nude mice. (Busson, P., et al., Establishment and characterization of three transplantable EBV-containing nasopharyngeal carcinomas. Int J Cancer, 42 : 599-606, 88.) It was established from the biopsy of a primary nasopharyngeal tumor in a 13-year-old girl born in Morocco. The biopsy was collected prior to any therapeutic procedure, according to the institutional guidelines concerning the use of clinical material. This patient had a voluminous primary tumor associated with lymph node and bone metastases and was treated by induction chemotherapy with a combination of vincristine, cyclophosphamide, doxorubicin and methylprednisolone, prior to nasopharyngeal and cervical radiotherapy. A 70% tumor response was achieved in both the primary tumor and cervical lymph node metastases after two months of chemotherapy. Complete clinical remission was obtained after radiotherapy, however remission lasted only 5 months, due to recurrence of bone metastases. C15 cells express a wild type p53 protein. (Effert, P., et al., Alterations of the p53 gene in nasopharyngeal carcinoma. J Virol, 66 : 3768-3775, 1992.)

C666-1 is an EBV-positive NPC cell line propagated *in vitro* related to an NPC xenograft, called xeno-666. (Cheung, S. T., et al., Nasopharyngeal carcinoma cell line (C666-1) consistently harbouring Epstein-Barr virus. Int J Cancer, 83 : 121-126, 1999.) A primary *in vitro* culture was derived from xeno-666 at passage 18 and named C666. Subsequently, C666 was adapted to low density growth and several subclones were isolated. One of them, named C666-1, was extensively characterized and later used in this study. (*Id*.; Weinrib, L., et al., Cisplatin chemotherapy plus adenoviral p53 gene therapy in EBV-positive and -negative nasopharyngeal carcinoma. Cancer Gene Ther, 8: 352-360., 2001.) C666-1 cells express a mutated p53 protein. (Weinrib, L., et al., Cancer Gene Ther, 8: 352-360., 2001.) In situ hybridization of EBER's (EBV-encoded RNA's).

Pieces of xenografted C15 and C666-1 tumors were fixed (in a mix of acetic acid, formaldehyde and ethanol), paraffin-embedded and cut in 4 µm sections. EBER's detection was performed by *in situ* hybridization with a mixture of peptide nucleic acid (PNA) probes reacting with both EBER 1 and 2 and labelled with fluorescein (Dako EBER-PNA probe, Dako, Trappes, France). The hybridization process was done as recommended by the manufacturer, using RNA's-free water. Hybridized probes were detected with anti-fluorescein antibodies conjugated to alcaline phosphatase (PNA ISH detection kit, Dako).

### Preparation of NPC cells for in vitro experiments.

Prior to *in vitro* experiments, C15 xenografted tumors were minced and treated with type II collagenase for cell dispersion, as previously reported. (Sbih-Lammali, F., et al., Control of apoptosis in Epstein Barr virus-positive nasopharyngeal carcinoma cells: opposite effects of CD95 and CD40 stimulation. Cancer Res, 59 : 924-930, 1999.) Residual cell aggregates were removed by filtration on a nylon cell strainer with 100 µm pores. C666-1 cells were permanently propagated *in vitro* in plastic flasks coated with collagene I (Biocoat, Becton-Dickinson, France). *In vitro* culture medium was Hepes-buffered RPMI with 5% fetal calf serum for both C15 and C666-1 cells. C666-1 cells which were derived from a single malignant clone were free of contaminating murine fibroblasts. In contrast, C15 cell suspensions were often contaminated by murine fibroblasts. Therefore, for some experiments, C15 cell suspensions were grown on plastic coated with Poly(2-HydroxyEthylMethacrylate), an anti-adhesive polymer which inhibits cell attachment, (Fukazawa, H., et al., Inhibitors of anchorage-independent growth affect the growth of transformed cells on poly(2-hydroxyethyl methacrylate)-coated surfaces. Int J Cancer, 67 : 876-882, 1996.), (PolyHEMA, Sigma, Saint-Quentin Fallavier, France). Using this coating procedure, fibroblast proliferation was completely inhibited whereas C15 cells grew as non-anchored spheroids or aggregates of 150 µm average diameter at 72 h (Fig. 1).

### Ki 67 immunostaining.

C15 cell aggregates were deposited on glass slides by cytospinning, fixed in acetone at 4°C for 10 min and stained with a mouse monoclonal antibody against the KI 67 antigen (Dako, Trappes, France). This antibody is specific of the human antigen and does not cross-react with its murine counter-part. Immunoreactivity was detected with peroxidase conjugated antibodies (Power vision kit, ImmunoVision technologies, Daly City, CA). Slides were counterstained with hematoxylin.

### In vitro prenyl-transferase assays.

The effect of Compound A and other drugs on FTase activity was assayed *in vitro* with FTase from human brain cytosol (ABS Reagents, Wilmington, DE) as target enzyme and recombinant human H-Ras protein containing the wild type CAAX box (Biomol, Plymouth Meeting, PA) as a specific substrate. The incubation mixture (25 µl) for [³H]-farnesylation contained 50 mM Tris-HCl (pH 7.5), 5 mM dithiothreitol, 20 µM ZnCl₂, 40 mM MgCl₂, 0.6 µM [³H]-farnesyl pyrophosphate (22.3 Ci /mmol) (NEN, Boston, MA), 4 µM recombinant H-Ras and 10 µg FTase. After 60 min at 37°C, the reaction was stopped by adding 150 µl of absolute ethanol. The mixture was then filtered on Unifilter GF/B microplate (Packard, Rungis, France) and washed 6 times with ethanol prior to scintillation counting. After adding 50µl of Microscint 0, plates were counted with a Packard Top Count scintillation counter. Drug effects on GGTase I activity were assayed by a similar method with GGTase I from human brain (ABS reagent) as target enzyme and human recombinant H-Ras containing a mutated CAAX box (CVLL)(Biomol). The incubation mixture contained 4 µM recombinant H-Ras, 0.6 µM [³H]-geranylgeranyl-pyrophosphate (19.3 Ci /mmol) (NEN) and 100 µg of GGTase I. GGTI-286 was used as a positive control for the GGTase-I inhibition assay. Results were expressed as the concentrations of drugs required to inhibit 50% of prenyl-incorporation into the recombinant H-Ras proteins (IC₅₀).

### Assessment of farnesyl-transferase inhibition in intact cells.

The effect of Compound A on farnesyl-transferase in intact cells was assayed in MIAPaca cells, a human pancreatic carcinoma cell line which was purchased from the ATCC. Both H-Ras and N-Ras were investigated as reference substrates of the endogenous farnesyl-transferase. Total protein extracts were prepared from MIAPaca cells treated for 48 h with increasing concentrations of Compound A (1-100 nM) along with extracts from untreated cells as a negative control. A positive control was provided by cells treated with mevastatine (30 µM). Mevastatine is an HMG-CoA reductase inhibitor which blocks the synthesis of farnesyl metabolic precursors. Cell extracts were separated by SDS-polyacrylamide gels (15%) and analyzed by Western blot with polyclonal antibodies directed to H-Ras and N-Ras (Santa Cruz, Heidelberg, Germany). Farnesyl-transferase inhibition was assessed indirectly by detection of non-prenylated forms of H-Ras and N-Ras which were slightly shifted toward higher molecular weights and reacted more efficiently with antibodies.

### Assessment of drug effect on NPC cell viability.

Cell viability assays were performed in 96 well plastic microplates, either uncoated (C666-1) or coated with Poly HEMA (C15). For each cell line, the number of cells distributed per well was optimized in order to achieve the highest metabolic activity while keeping exponential growth till the end of the test. C15 and C666-1 cells were seeded at 10⁵ and 35 X 10³/well, respectively, in 150 µl culture medium. Following an overnight pre-incubation culture, serial dilutions of chemotherapy drugs (50 µl; final concentration 50 nM - 50 µM) were added in quadruplicate to a final volume of 250 µl. At the completion of a 72 h incubation with tested pharmacological agents, cell viability was evaluated using the WST-1 assay (Roche Molecular, France). The WST-1 assay is based on the cleavage of the tetrazolium salt by mitochondrial dehydrogenases. In contrast with other tests, it does not require washing the cells in microplate wells and is compatible with spheroid culture. Cells were incubated with 10 µl of the WST-1 reagent added to the culture medium, for 3 to 6 h, at 37°C. The plates were subsequently read on an ELISA reader (Dynatech MR7000, Guernsey channel Island, USA) using a 490 nm filter. The mean and standard deviation were determined for quadruplicate samples. For each compound, values falling in the linear part of sigmoid curve were included in a linear regression analysis and were used to estimate the 50% inhibitory concentration (IC 50).

### Measurement of caspase activity in drug treated NPC cells.

Caspase activity was assessed at single cell level by flow cytometry using the CaspaTag kit according to manufacturer instructions (Quantum-Appligene, Illkirch, France). This procedure involved a cell-permeable, general caspase inhibitor (VAD-fluoromethyl ketone) labelled with carboxyfluorescein. This fluorescent inhibitor irreversibly binds to active caspases and therefore is selectively retained in apoptotic cells. For apoptosis induction, C15 and C666-1 cells were seeded in 24 well plates at 10⁶ and 3 x 10⁵ cells/well respectively, pre-incubated overnight and treated with drugs for 48 h. In this context, it was not possible to incubate C15 cells on PolyHEMA, because the resulting cell aggregates were not suitable for flow cytometry. In order to discriminate human malignant cells from contaminating murine fibroblasts, C15 cell suspensions were submitted to additional staining with an anti-human HLA (human leukocyte antigen) A, B, C directly conjugated to allo-Phyco-Cyanine (Becton Dickinson, Meylan, France). In a first step, both C15 and C666-1 cells were incubated for 1 h at 37°C, in their culture plates, with fluorescent VAD-FMK, in 300 µl of culture medium. Cells were then washed and trypsinized. C15 cells were further incubated with the anti-HLA antibody. Finally, both caspase and HLA fluorescence were analysed using a FACS calibur flow cytometer (Becton Dickinson, Franklin Lakes, NJ).

### Assessment ofPARP (Poly(ADP-ribose)polymerase) and TRAF1 cleavage.

Whole cell extracts were prepared from drug-treated and control C15 and C666-1 cells in RIPA-SDS buffer. (Sbih-Lammali, F., et al., Control of apoptosis in Epstein Barr virus-positive nasopharyngeal carcinoma cells: opposite effects of CD95 and CD40 stimulation. Cancer Res, 59:924-930, 1999.) Thirty to fifty µg of total protein extract were submitted to electrophoresis on 7.5% (PARP) or 8-16% gradient (TRAF1) SDS-polyacrylamide gels. Separated proteins were transferred to Immobilon membranes (Millipore, France) which were probed with anti-PARP (Oncogene, Boston, MA) or anti-TRAF-1 (Santa Cruz, Heidelberg, Germany) antibodies revealed with horseradish peroxidase-conjugated antibodies (Amersham, Les Ulis, France). Detection was performed with the ECL chemiluminescence system (Amersham, Les Ulis, France). Two types of antibodies from Santa Cruz were used for study of TRAF1-cleavage: the monoclonal H-3 and the H-132 polyclonal; a positive control was provided by C15 cells treated for 24 h with the CD95-agonist antibody, 7C11 (Immmunotech, Marseille, France).

### Detection of EBER's in NPC tumor lines.

In order to check that both C15 and C666-1 retained latent EBV-infection, EBER's expression was detected by *in situ* hybridization in both tumor lines (Fig. 1 A and B) (C666-1 xenografted tumors were reformed by cell injection into nude mice at *in vitro* passage 40). As expected, EBER's staining was essentially nuclear. Most but not all malignant cells were positively stained, an observation which is consistent with previous reports about fresh NPC biopsies. (Wu, T. C., et al., Abundant expression of EBER1 small nuclear RNA in nasopharyngeal carcinoma. A morphologically distinctive target for detection of Epstein-Barr virus in formalin-fixed paraffin-embedded carcinoma specimens. Am J Pathol, 138: 1461-1469, 1991.)

### Assessment of C15 and C666-1 proliferation.

As previously reported, C666-1 cells consistently proliferated *in vitro,* growing as cell monolayers in various types of plastic vessels. (Cheung, S. T., et al., Nasopharyngeal carcinoma cell line (C666-1) consistently harbouring Epstein-Barr virus. Int J Cancer, 83 : 121-126, 1999.) In order to prevent increasing contamination by murine fibroblasts, tumor-dispersed C15 cells were seeded in microplates coated with PolyHema matrix and grown as floating aggregates. These aggregated cells remained proliferating as demonstrated by immuno-cytological detection of the human Ki-67 antigen in a significant fraction of them (Fig. 1C). In addition, repeated measurements of WST reduction during 3 consecutive days of culture demonstrated a consistent, steady increase of viable cells (Fig. 1D). C15 cell doubling time was estimated at 1.5 days. In the same plates, without plastic coating, the doubling time of C666-1 cells was about 3.5 days (Fig. 1D).

### Short term cytotoxicity of conventional drugs applied on NPC cells in vitro.

Cis-platinum, bleomycine, 5FU, doxorubicine and taxol are among the drugs most frequently used in chemotherapy of NPC. (Ali, H. et al., Chemotherapy in advanced nasopharyngeal cancer. Oncology (Huntingt), 14:1223-1230, 2000.) Their short term cytotoxic effect was assessed *in vitro* on the C15 and C666-1 cells, using a cell viability assay based on WST reduction. Cultured cells were incubated in the presence of various concentrations of each therapeutic agent for 72 hours. As shown Table I, both C15 and C666-1 were highly sensitive to doxorubicin at concentrations of below 1 µM. C666-1 cells were also highly sensitive to taxol, whereas C15 cells were totally resistant to this drug. On the other hand, C15 was mildly sensitive to the cytotoxic effect of cis-platinum (1 µM IC₅₀) whereas C666-1 cells were five times more resistant. No significant effects of bleomycin or 5-FU were observed in either cell line at the concentrations tested.

### Characterization of a farnesyl-transferase inhibitor

Although doxorubicin was very active on both C15 and C666-1, it was not observed to induce massive apoptosis in these cell lines at the concentrations tested. Therefore it was postulated to use molecularly targeted agents, especially farnesyl-transferase inhibitors (FTI's) in combination with doxorubicin, to increase its cytotoxic effect and attempt to induce apoptosis. Compound A is a peptidomimetic FTI which had been designed to be highly selective of farnesyl-transferase. The biological activity of this compound was first assayed for its effect on the activity of purified human prenyl-transferase and its inhibition of ras-processing in intact cells. Table II shows that Compound A is a potent inhibitor of human brain FTase *in vitro*. The IC₅₀ value is in the nanomolar range and compares favourably with the tested known FTI compounds FTI-277 and BIM-46068. (Sun, J., et al., Ras CAAX peptidomimetic FTI 276 selectively blocks tumor growth in nude mice of a human lung carcinoma with K-Ras mutation and p53 deletion. Cancer Res, 55 : 4243-4247, 1995; Prevost, G. P., et al., Inhibition of human tumor cell growth in vitro and in vivo by a specific inhibitor of human farnesyltransferase: BIM-46068. Int J Cancer, 83 : 283-287, 1999.) It is noteworthy that in contrast to other tested FTI's, no activity on GGTase-I by Compound A was observed at concentrations of up to 100 µM thus showing its high selectivity for FTase. The effect of Compound A on protein-farnesylation was further assessed in intact MIAPaca cells which are prototype target cells for prenylation-inhibitors. H-Ras and N-Ras, which are classical substrates for farnesyl-transferases, are both expressed and non-mutated in MIAPaca cells. A significant inhibition of H-Ras and N-Ras farnesylation was obtained when cells were incubated with only 50 nM of Compound A for 48 h.

### Enhancement of doxorubicin cytotoxic activity on NPC cells when used in combination with Compound A.

As shown in Figure 2A, Compound A had only limited toxicity on both C15 and C666-1 cells when it was used alone (IC₅₀= 10 µM). However, the cytotoxic effect of doxorubicin was dramatically enhanced when it was combined with Compound A. For C15 cells, a more than additive effect was obvious for doxorubicin concentrations of 500 nM or 1 µM with Compound A at 5 µM. For C666-1 cells a higher concentration of the FTI drug was required to obtain a synergistic effect with doxorubicin. Still, a more than additive effect was observed for doxorubicin concentrations of 1 and 2 µM. In contrast, enhanced cisplatin and bleomycin cytotoxicity was not observed by combination with Compound A at test concentrations.

### Contribution of apoptosis to the cytotoxic effect of the doxorubicin/Compound A combination.

After 48 h incubation with the doxorubicin/Compound A combination, many C15 cells started to round up, retract their processes, and subsequently detach from culture dishes. Under nuclear staining with Hoechst 33342, typical changes related to nuclear apoptosis - nuclear condensation and fragmentation - were detected at 48 h of incubation and became more obvious after 72 h. Such morphological changes were much less apparent in C15 cells treated with doxorubicin or Compound A alone. In contrast to C15 cells, significant changes in the morphology of the nucleus were not observed in C666-1 cells, even in the presence of both doxorubicin and Compound A, although some nuclei with partial chromatin condensation were recorded. Apoptosis of C15 cells under treatment by both drugs was further demonstrated by flow cytometry analysis of caspase activation which was obvious after a 48 h period of treatment. Finally PARP cleavage was analysed by Western blot in protein extracts of C15 cells treated by one drug or the association of both. A strong PARP cleavage was apparent after only a 24 h period of combined treatment. At 48 h, the intact fragment of the PARP was almost undetectable. Moderate PARP cleavage was also detected in cells treated by doxorubicin or Compound A alone, but a much lower fraction of the protein was affected in these experimental conditions. No caspase-activation and PARP cleavage were detected in C666-1 cells even when treated by doxorubicin combined with Compound A.

### Cleavage of TRAF1 in C15 cells treated by the Compound A-doxorubicin combination.

NPC cells treated by doxorubicin and/or Compound A were investigated for TRAF1-cleavage. TRAF1 is a signalling adapter which has a restricted tissue distribution but whose expression is ectopically induced by EBV-infection. (Mosialos, G., et al., The Epstein-Barr virus transforming protein LMP1 engages signaling proteins for the tumor necrosis factor receptor family. Cell, 80 : 389-399, 1995.) It has been reported that TRAF1 is strongly expressed by EBV-positive NPC cells. (Ardila-Osorio, H., et al., Evidence of LMP1-TRAF3 interactions in glycosphingolipid-rich complexes of lymphoblastoid and nasopharyngeal carcinoma cells. Int J Cancer, 81 : 645-649, 1999.) On the other hand, Leo et al. (2001) have reported that TRAF1 is cleaved at aspartate 163 in cells undergoing apoptosis, especially death receptor-mediated apoptosis but also doxorubicin-induced apoptosis. (Leo, E., et al., TRAF1 is a substrate of caspases activated during tumor necrosis factor receptor-α induced apoptosis. J Biol Chem, 55: 8087-8093, 2000) C15 cells treated by a CD95-agonist (7C11) for 24 h were used as a positive control; a dramatic decrease in the amount of intact TRAF1 was observed in the corresponding protein extracts. Simultaneously, there was a marked increase of the cleaved fragments characterised by Leo et al., (*Id*.): fragment I (24 kD, reacting with the H-132 antibody) and II (30kD, reacting with H-3). In cells treated with doxorubicin or Compound A alone, no modifications of the TRAF1 molecule were observed. In contrast, a substantial increase of cleaved fragments similar to fragments I and II were observed in the extracts of cells treated by the combination of doxorubicin and Compound A. There were some differences in the patterns of cleavage induced by the CD95-agonist and the drug combination. Despite the visualisation of cleaved fragments, the amount of intact TRAF1 molecule was only marginally reduced in drug-treated cells. Further the cleaved fragment II consistently had a slightly bigger size than with the 7C11 agonist. TRAF1 was also detected in C666-1 cells and, although readily cleaved under treatment by the CD95-agonist, it was not cleaved under drug-treatment. This observation is consistent with the absence of apoptotic process.

Unless defined otherwise, all technical and scientific terms used herein have the same meaning as commonly understood by one of ordinary skill in the art to which this invention belongs.

**Table I : Effects of conventional drugs on NPC cell viability in vitro. Determination of the IC 50.**

| | C15 Cells | C666-1 Cells |
|---|---|---|
| Doxorubicin | 150 nM | 200 nM |
| Cis-platinum | 1 µM | 5 µM |
| Bleomycin | 5 µM | 5 µM |
| 5-fluorouracyl | 5 µM | 5 µM |
| Taxol | 25 µM | 200 nM |

| | | |
|---|---|---|
| - Data are the mean of quadruplicates. Similar results were obtained in three separate experiments. | | |

**Table II: Comparative assessment of FTase inhibitor action on purified human prenyl transferase activities.**

| | Enzyme assays IC₅₀ (nM) | |
|---|---|---|
| | FTase | GGTase I |
| *BIM-46068* | 91 (83- 99) | 268000 (15200-384000) |
| *FTI-277* | 30 (26- 34) | 314 (305- 323) |
| *GGTI-286* | 365 (329- 401) | 114 (106- 123) |
| *Compound A* | 15 (13 - 15) | > 100 µM |

| | | |
|---|---|---|
| - Assay results are reported as the mean of 2 experiments with the lowest and highest IC₅₀ values observed in individual experiments in parentheses. | | |

## Claims

1. A pharmaceutical composition comprising a farnesyl transferase inhibitor, or a pharmaceutically acceptable salt thereof and an anthracycline, or a pharmaceutically acceptable salt thereof, wherein said farnesyl transferase inhibitor is according to formula I: 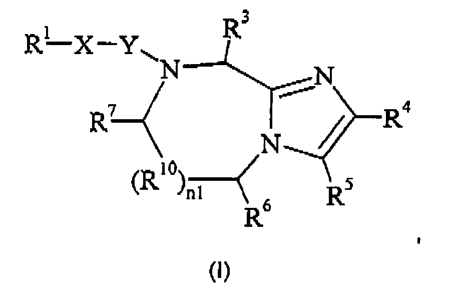 wherein
n1 is 1;
X is, independently for each occurrence, (CHR¹¹)ₙ₃(CH₂)ₙ₄Z(CH₂)ₙ₅;
Z is a bond;
n3 is, independently for each occurrence, 0 or 1;
n4 and n5 each is, independently for each occurrence, 0, 1, 2, or 3;
Y is, independently for each occurrence, CO;
R¹ is 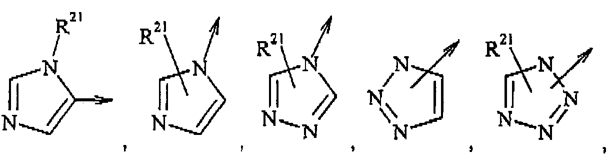 or 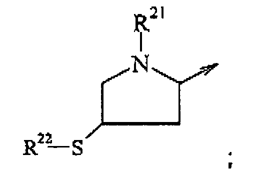
R² and R¹² each is, independently for each occurrence, H or an optionally substituted moiety selected from the group consisting of (C₁₋₆)alkyl and aryl, wherein said optionally substituted moiety is optionally substituted with one or more of R⁶ or R³⁰;
R¹¹ is, independently for each occurrence, H;
R³ is, independently for each occurrence, H or an optionally substituted moiety selected from the group consisting of (C₁₋₆)alkyl, (C₂₋₆)alkenyl, (C₂₋₆)alkynyl, (C₃₋₆)cycloalkyl, (C₃₋₆)cycloalkyl(C₁₋₆)alkyl, (C₅₋₇)cycloalkenyl, (C₅₋₇)cycloalkenyl(C₁₋₆)alkyl, aryl, aryl(C₁₋₆)alkyl, heterocyclyl, and heterocyclyl(C₁₋₆)alkyl, wherein said optionally substituted moiety is optionally substituted with one or more R³⁰;
R⁴ is, independently for each occurrence, an optionally substituted phenyl moiety, wherein said optionally substituted phenyl moiety is optionally substituted with one or more R³⁰, wherein each said substituent is independently selected;
R⁵ is, independently for each occurrence, H;
R⁶ is, independently for each occurrence, H or an optionally substituted moiety selected from the group consisting of (C₁₋₆)alkyl, (C₂₋₆)alkenyl, (C₃₋₆)cycloalkyl, (C₃₋₆)cycloalkyl(C₁₋₆)alkyl, (C₅₋₇)cycloalkenyl, (C₅₋₇)cycloalkenyl(C₁₋₆)alkyl, aryl, aryl(C₁₋₆)alkyl, heterocyclyl, and heterocyclyl(C₁₋₆)alkyl, wherein said optionally substituted moiety is optionally substituted with one or more substituents each independently selected from the group consisting of OH, (C₁₋₆)alkyl, (C₁₋₆)alkoxy, -N(R⁶R⁹), -COOH, -CON(R⁸R⁹), and halo, where R⁸ and R⁹ each is, independently for each occurrence, H, (C₁₋₆)alkyl, (C₂₋₆)alkenyl, (C₂₋₆)alkynyl, aryl, or aryl(C₁₋₆)alkyl;
R⁷ is, independently for each occurrence, H, =O, =S, or an optionally substituted moiety selected from the group consisting of (C₁₋₆)alkyl, (C₂₋₆)alkenyl, (C₃₋₆)cycloalkyl, (C₃₋₆)cycloalkyl(C₁₋₆)alkyl, (C₅₋₇)cycloalkenyl, (C₅₋₇)cycloalkenyl(C₁₋₆)alkyl, aryl, aryl(C₁₋₆)alkyl, heterocyclyl, and heterocyclyl(C₁₋₆)alkyl, wherein said optionally substituted moiety is optionally substituted with one or more substituents each independently selected from the group consisting of OH, (C₁₋₆)alkyl, (C₁₋₆)alkoxy, -N(R⁸R⁹), -COOH, -CON(R⁸R⁹), and halo;
R¹⁰ is C;
R²¹ is, independently for each occurrence, H or an optionally substituted moiety selected from the group consisting of (C₁₋₆)alkyl and aryl(C₁₋₆)alkyl, wherein said optionally substituted moiety is optionally substituted with one or more substituents each independently selected from the group consisting of R⁸ and R³⁰;
R²² is H, (C₁₋₆)alkylthio, (C₃₋₆)cycloalkylthio, R⁸-CO-, or a substituent according to the formula 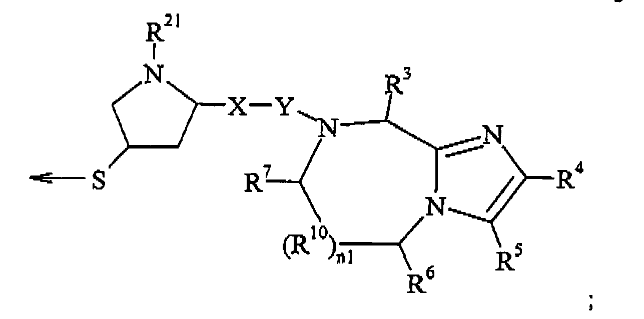
R²⁴ and R²⁵ each is, independently for each occurrence, H, (C₁₋₆)alkyl, or aryl(C₁₋₆)alkyl;
R³⁰ is, independently for each occurrence, (C₁₋₆)alkyl, -O-R⁶, -S(O)ₙ₆R⁸, - S(O)ₙ₇N(R⁸R⁹), -N(R⁸R⁹), -CN, -NO₂, -CO₂R⁸, -CON(R⁸R⁹), -NCO-R⁸, or halogen; n6 and n7 each is, independently for each occurrence, 0, 1, or 2;
wherein said heterocyclyl is azepinyl, benzimidazolyl, benzisoxazolyl, benzofurazanyl, benzopyranyl, benzothiopyranyl, benzofuryl, benzothiezolyl, benzothienyl, benzoxazolyl, chromanyl, cinnolinyl, dihydrobenzofuryl, dihydrobenzothienyl, dihydrobenzothiopyranyl, dihydrobenzothio-pyranyl sulfone, furyl, imidazolidinyl, imidazolinyl, imidazolyl, indolinyl, indolyl, isochromanyl, isoindolinyl, isoquinolinyl, isothiazolidinyl, isothiazolyl, isothiazolidinyl, morpholinyl, naphthyridinyl, oxadiazolyl, 2-oxoazepinyl, 2-oxopiperazinyl, 2-oxopiperidinyl, 2-oxopyrrolidinyl, piperidyl, piperazinyl, pyridyl, pyridyl N-oxide, quinoxalinyl, tetrahydrofuryl, tetrahydroisoquinolinyl, tetrahydro-quinolinyl, thiamorpholinyl, thiamorpholinyl sulfoxide, thiazolyl, thiazolinyl, thienofuryl, thienothienyl, or thienyl; and
wherein said aryl is phenyl or naphthyl;
provided that:
R⁶ is H, and R¹⁰ and R⁷ are taken together to form 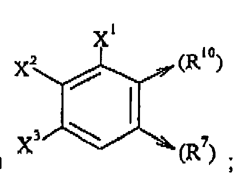 or
R⁷ is =O, -H, or =S, and R¹⁰ and R⁶ are taken together to form 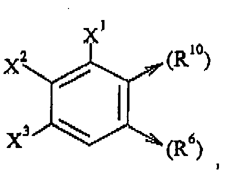 wherein X¹, X², and X³ each is, independently, H, halogen, -NO₂, -NCO-R⁸, - CO₂R⁸, -CN, or -CON(R⁸R⁹); or a pharmaceutically acceptable salt thereof.

2. A pharmaceutical composition according to claim 1, wherein:
R¹ is 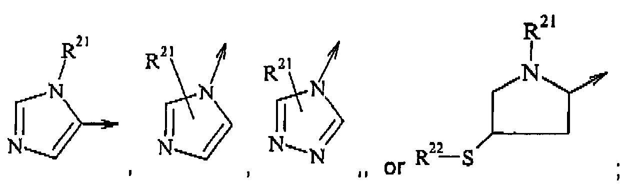 and
X is CH(R¹¹)ₙ₃(CH₂)ₙ₄ ;
or a pharmaceutically acceptable salt thereof.

3. A pharmaceutical composition according to claim 2, wherein:
R¹ is 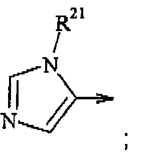
R⁶ is H;
n1 is 1;
R⁷ and R¹⁰ are taken together to form 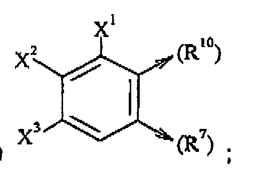
n3 is 1 and R¹¹ is H;
Z is a bond;
n5 is 0; and
Y is CO;
or a pharmaceutically acceptable salt thereof.

4. A pharmaceutical composition according to claim 2, wherein said farnesyl transferase inhibitor is a compound of formula I, wherein:
R¹ is 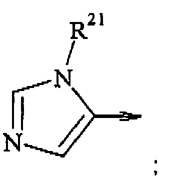
R⁷ is H or =O;
n1 is 1;
R⁶ and R¹⁰ are taken together to form 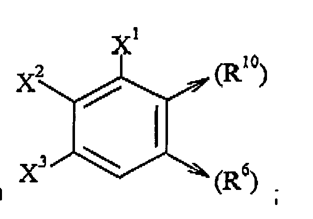
n3 is 1 and R¹¹ is H;
n5 is 0;
Y is CO ; and
Z is a bond;
or a pharmaceutically acceptable salt thereof.

5. A pharmaceutical composition according to claim 1, wherein said farnesyl transferase inhibitor is:
1-(2-(1-(4-cyanophenylmethyl)imidazol-4-yl)-1-oxoethyl)-1,2-dihydro-4-(2-methoxyphenyl)-imidazo[1,2-c][1,4]benzodiazepine ;
9-bromo-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
9-Chloro-1-(2-(1-(4-cyanophenylmethyl)imidazol-yl)-1-oxoethyl)-1,2-dihydro-4-(2-methoxyphenyl)-imidazo[1,2-c][1,4]benzodiazepine;
10-Bromo-1-(2-(1-(4-cyanophenylmethyl)imidazol-yl)-1-oxoethyl)-1,2-dihydro-4-(2-methoxyphenyl)-imidazo[1,2-c][1,4]benzodiazepine;
1-(2-(1-(4-cyanophenylmethyl)imidazol-4-yl)-1-oxoethyl)-1,2-dihydro-8-fluoro-4-(2-methoxyphenyl)-imidazo[1,2-c][1,4]benzodiazepine; or
or a pharmaceutically acceptable salt thereof.

6. A pharmaceutical composition according to claim 9, wherein said farnesyl transferase inhibitor is:
1-(2-(1-(4-cyanophenylmethyl)imidazol-4-yl)-1-oxoethyl)-1,2-dihydro-4-(2-methoxyphenyl)-imidazo[1,2-c][1,4]benzodiazepine ;
9-bromo-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
9-Chloro-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
10-Bromo-1-(2-(1-(4-cyanophenylmethyl)imidazol-4-yl)-1-oxoethyl)-1,2-dihydro-4-(2-methoxyphenyl)-imidazo[1,2-c][1,4]benzodiazepine;
1-(2-(1-(4-cyanophenylmethyl)imidazol-4-yl)-1-oxoethyl)-1,2-dihydro-8-fluoro-4-(2-methoxyphenyl)-imidazo[1,2-c][1,4]benzodiazepine;

7. A pharmaceutical composition according to claim 1, wherein said farnesyl transferase inhibitor is:
5-(2-(1-(4-cyanophenylmethyl)-imidazol-5-yl)-1-oxo-ethyl)-5,6-dihydro-2-phenyl-1H-imidazo[1,2-a][1,4]benzodiazepine;
or a pharmaceutically acceptable salt thereof.

8. A pharmaceutical composition according to claim 1, wherein said farnesyl transferase inhibitor is:
1,2-dihydro-1-(2-(imidazol-1-yl)-1-oxoethyl)-4-(2-methoxyphenyl) imidazo[1,2a][1,4]benzodiazepine;
or a pharmaceutically acceptable salt thereof.

9. A pharmaceutical composition according to claim 1, wherein said farnesyl transferase inhibitor is: 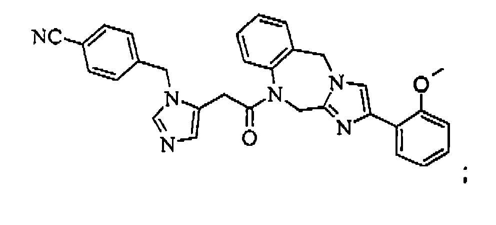 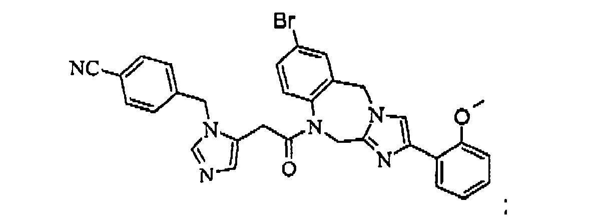 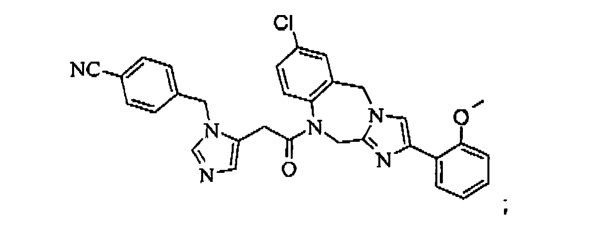 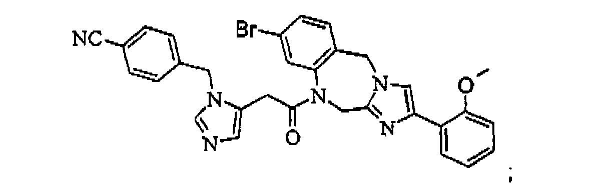 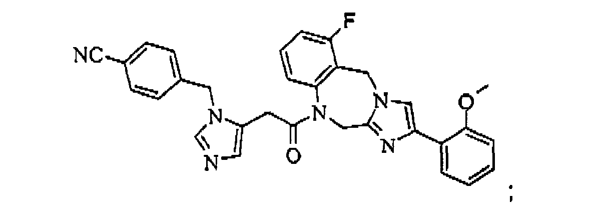 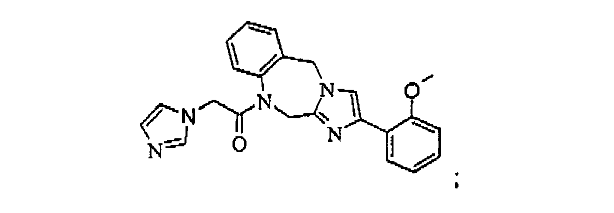 or 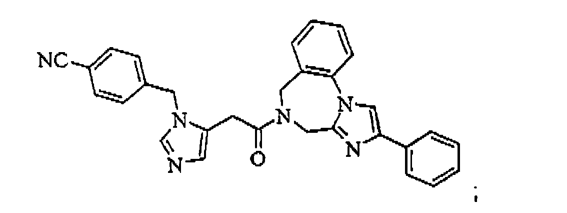 or a pharmaceutically acceptable salt thereof.

10. A pharmaceutical composition according to claim 1, wherein said farnesyl transferase inhibitor is: 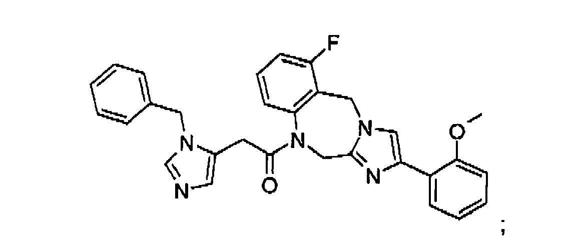 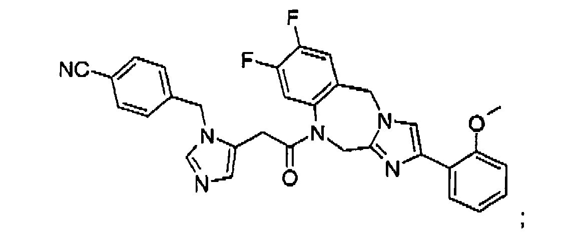 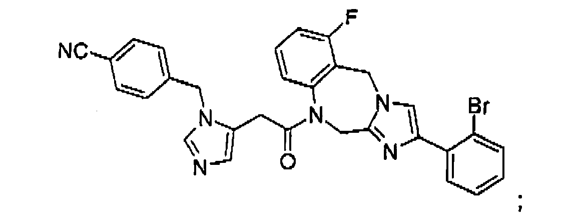 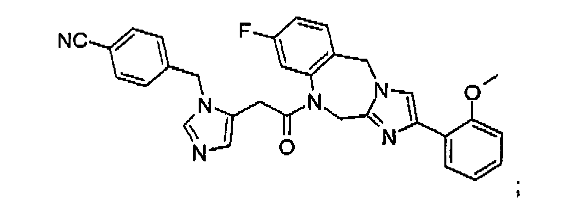 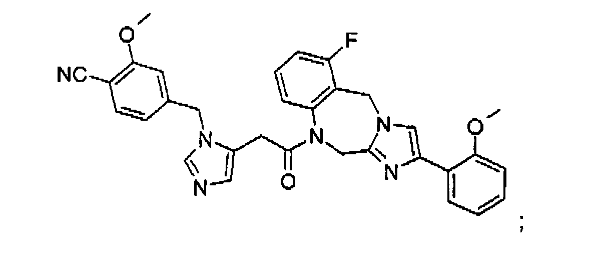 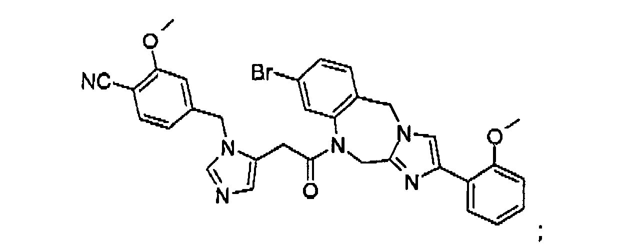 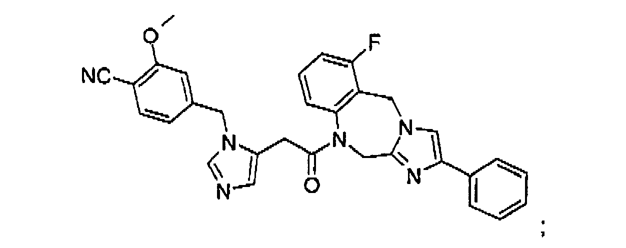 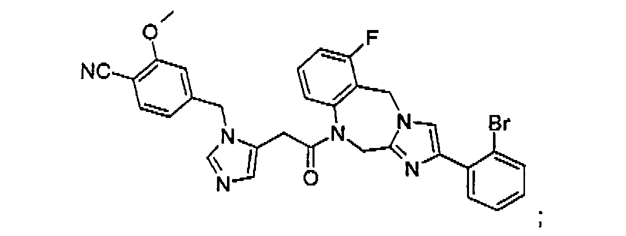 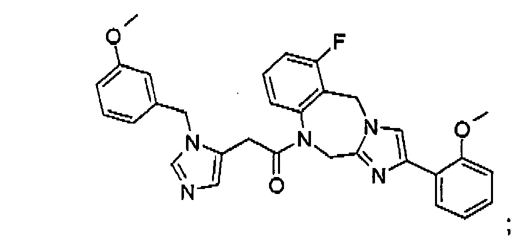 or 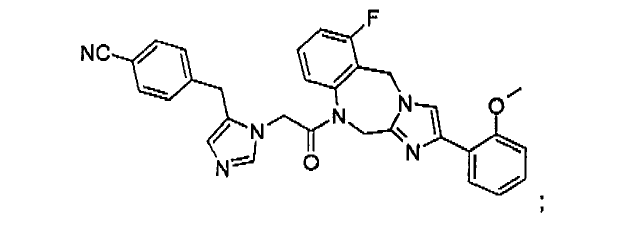 or a pharmaceutically acceptable salt thereof.

11. A pharmaceutical composition according to claim 10, wherein said farnesyl transferase inhibitor is: 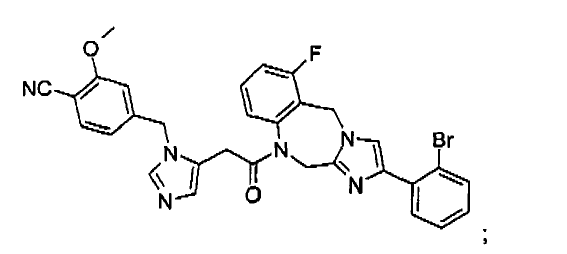 or a pharmaceutically acceptable salt thereof.

12. A pharmaceutical composition according to claim 11, wherein said anthracycline is doxorubicin, daunorubicin, epirubicin, idarubicin, or amrubicin, or a pharmaceutically acceptable salt thereof.

13. A pharmaceutical composition according to claim 11, wherein said anthracycline is doxorubicin, or a pharmaceutically acceptable salt thereof.

14. A pharmaceutical composition according to any one of claims 1 - 11, wherein said anthracycline is doxorubicin, daunorubicin, epirubicin, idarubicin, or amrubicin, or a pharmaceutically acceptable salt of said anthracycline.

15. A pharmaceutical composition according to claim 14, wherein said anthracycline is doxorubicin, or a pharmaceutically acceptable salt thereof.

16. An in vitro method of decreasing the rate of proliferation of nasopharyngeal carcinoma cells, said method comprising contacting said nasopharyngeal cells with a pharmaceutical composition according to any one of claims 12 to 15.

17. Use of a pharmaceutical composition according to any one of claims 12 to 15 in the preparation of a medicament for treating nasopharyngeal carcinoma in a patient.

18. Use of an effective amount of one or more farnesyl transferase inhibitors according to claim 1 in combination with an effective amount of one or more anthracycline, in the preparation of a medicament for treating nasopharyngeal carcinoma in a patient, wherein said effective amount of said farnesyl transferase inhibitor and of said anthracycline are effective in combination to treat said nasopharyngeal carcinoma.

19. Use according to claim 18 wherein said patient is a mammal.

20. Use according to claim 19 wherein said patient is a human being.

21. Use according to claim 20 wherein said farnesyl transferase inhibitor and said anthracycline are administered substantially simultaneously.

22. A pharmaceutical kit comprising a composition according to any one of claims 12 to 15 and instructions for use of said composition for the treatment of nasopharyngeal carcinoma.

23. A kit comprising: a) a first unit dosage form comprising a farnesyl transferase inhibitor according to claim 1 or a pharmaceutically acceptable salt thereof and a pharmaceutically acceptable carrier, vehicle or diluent; b) a second unit dosage form comprising an anthracycline, or a pharmaceutically acceptable salt thereof and a pharmaceutically acceptable carrier, vehicle or diluent; and c) a container.

24. A kit according to claim 23, wherein said farnesyl transferase inhibitor comprises a compound according to formula I or a pharmaceutically acceptable salt thereof, and said anthracycline comprises doxorubicin, daunorubicin, epirubicin, idarubicin, or amrubicin or a pharmaceutically acceptable salt thereof.

25. A kit according to claim 24, wherein said farnesyl transferase inhibitor comprises a compound according to the formula: 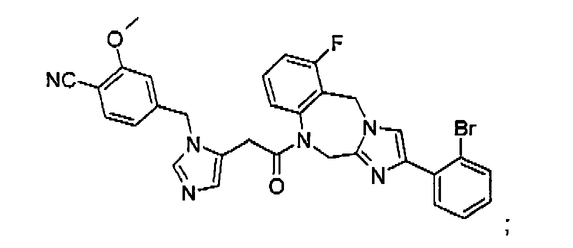 or a pharmaceutically acceptable salt thereof, and said anthracycline comprises doxorubicin or a pharmaceutically acceptable salt thereof.

## Patentansprüche

1. Pharmazeutische Zusammensetzung, die einen Farnesyltransferaseinhibitor oder ein pharmazeutisch annehmbares Salz davon und ein Anthracyclin oder ein pharmazeutisch annehmbares Salz davon umfasst, wobei der Farnesyltransferaseinhibitor die Formel I: 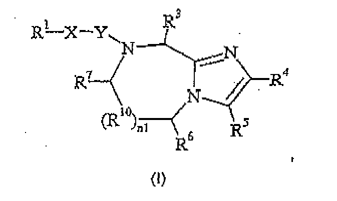 aufweist, in der
n1 1 ist,
X unabhängig bei jedem Vorkommen (CHR¹¹)ₙ₃(CH₂)ₙ₄Z(CH₂)ₙ₅ ist,.
Z eine Bindung ist,
n3 unabhängig bei jedem Vorkommen 0 oder 1 ist,
n4 und n5 jeweils unabhängig bei jedem Vorkommen 0, 1, 2 oder 3 sind,
Y unabhängig bei jedem Vorkommen CO ist, 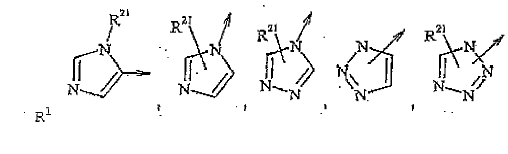 oder 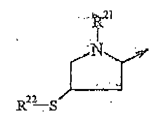 ist,
R² und R¹² jeweils unabhängig bei jedem Vorkommen H oder ein gegebenenfalls substituierter Anteil ausgewählt aus der Gruppe bestehend aus (C₁₋₆)-Alkyl und Aryl sind, wobei der gegebenenfalls substituierte Anteil gegebenenfalls mit einem oder mehreren von R⁸ oder R³⁰ substituiert ist,
R¹¹ unabhängig bei jedem Vorkommen H ist,
R³ unabhängig bei jedem Vorkommen H oder ein gegebenenfalls substituierter Anteil ausgewählt aus der Gruppe bestehend aus (C₁₋₆)-Alkyl, (C₂₋₆)-Alkenyl, (C₂₋₆)-Alkinyl, (C₃₋₆)-Cycloalkyl, (C₃₋₆)-Cycloalkyl-(C₁₋₆)-alkyl, (C₅₋₇)-Cycloalkenyl, (C₅₋₇)-Cycloalkenyl-(C₁₋₆)-alkyl, Aryl, Aryl-(C₁₋₆)-alkyl, Heterocyclyl und Heterocyclyl-(C₁₋₆)-alkyl ist, wobei der gegebenenfalls substituierte Anteil gegebenenfalls mit einem oder mehreren R³⁰ substituiert ist,
R⁴ unabhängig bei jedem Vorkommen ein gegebenenfalls substituierter Phenylanteil ist, wobei der gegebenenfalls substituierte Phenylanteil gegebenenfalls mit einem oder mehreren R³⁰ substituiert ist, wobei jeder Substituent unabhängig ausgewählt ist,
R⁵ unabhängig bei jedem Vorkommen H ist,
R⁶ unabhängig bei jedem Vorkommen H oder ein gegebenenfalls substituierter Anteil ausgewählt aus der Gruppe bestehend aus (C₁₋₆)-Alkyl, (C₂₋₆)-Alkenyl, (C₃₋₆)-Cycloalkyl, (C₃₋₆)-Cycloalkyl-(C₁₋₆)-alkyl, (C₅₋₇)-Cycloalkenyl, (C₅₋₇)-Cycloalkenyl-(C₁₋₆)-alkyl, Aryl, Aryl-(C₁₋₆)-alkyl, Heterocyclyl und Heterocyclyl-(C₁₋₆)-alkyl ist, wobei der gegebenenfalls substituierte Anteil gegebenenfalls mit einem oder mehreren Substituenten substituiert ist, die jeweils unabhängig ausgewählt sind aus der Gruppe bestehend aus OH, (C₁₋₆)-Alkyl, (C₁₋₆)-Alkoxy, -N(R⁸R⁹), -COOH, -CON(R⁸R⁹) und Halogen, wobei R⁸ und R⁹ jeweils unabhängig bei jedem Vorkommen H, (C₁₋₆)-Alkyl, (C₂₋₆)-Alkenyl, (C₂₋₆)-Alkinyl, Aryl oder Aryl-(C₁₋₆)-alkyl ist,
R⁷ unabhängig bei jedem Vorkommen H, =O, =S oder ein gegebenenfalls substituierter Anteil ausgewählt aus der Gruppe bestehend aus (C₁₋₆)-Alkyl, (C₂₋₆)-Alkenyl, (C₃₋₆)-Cycloalkyl, (C₃₋₆)-Cycloalkyl-(C₁₋₆)-alkyl, (C₅₋₇)-Cycloalkenyl, (C₅₋₇)-Cycloalkenyl-(C₁₋₆)-alkyl, Aryl, Aryl-(C₁₋₆)-alkyl, Heterocyclyl und Heterocyclyl-(C₁₋₆)-alkyl ist, wobei der gegebenenfalls substituierte Anteil gegebenenfalls mit einem oder mehreren Substituenten substituiert ist, die jeweils unabhängig ausgewählt sind aus der Gruppe bestehend aus OH, (C₁₋₆)-Alkyl, (C₁₋₆)-Alkoxy, -N(R⁸R⁹), -COOH, -CON(R⁸R⁹) und Halogen,
R¹⁰ C ist,
R²¹ unabhängig bei jedem Vorkommen H oder ein gegebenenfalls substituierter Anteil ausgewählt aus der Gruppe bestehend aus (C₁₋₆)-Alkyl und Aryl-(C₁₋₆)-alkyl ist, wobei der gegebenenfalls substituierte Anteil gegebenenfalls mit einem oder mehreren Substituenten substituiert ist, die jeweils unabhängig aus der Gruppe bestehend aus R⁸ und R³⁰ ausgewählt sind,
R²² H, (C₁₋₆)-Alkylthio, (C₃₋₆)-Cycloalkylthio, R⁶-CO- oder ein Substituent mit der folgenden Formel ist: 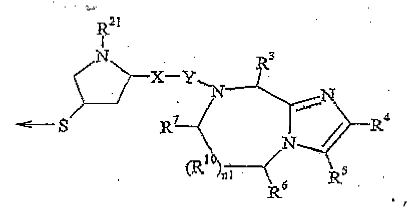
R²⁴ und R²⁵ jeweils unabhängig bei jedem Vorkommen H, (C₁₋₆)-Alkyl oder Aryl-(C₁₋₆)-alkyl sind,
R³⁰ unabhängig bei jedem Vorkommen (C₁₋₆)-Alkyl, -O-R⁸, -S(O)ₙ₆R⁸, -S(O)ₙ₇N(R⁸R⁹), -N(R⁸R⁹), -CN, -NO₂, -CO₂R⁸, -CON(R⁸R⁹), -NCO-R⁸ oder Halogen ist,
n6 und n7 jeweils unabhängig bei jedem Vorkommen 0, 1 oder 2 sind,
wobei das Heterocyclyl Azepinyl, Benzimidazolyl, Benzisoxazolyl, Benzofurazanyl, Benzopyranyl, Benzothiopyranyl, Benzofuryl, Benzothiazolyl, Benzothienyl, Benzoxazolyl, Chromanyl, Cinnolinyl, Dihydrobenzofuryl, Dihydrobenzothienyl, Dihydrobenzothiopyranyl, Dihydrobenzothiopyranylsulfon, Furyl, Imidazolidinyl, Imidazolinyl, Imidazolyl, Indolinyl, Indolyl, Isochromanyl, Isoindolinyl, Isochinolinyl, Isothiazolidinyl, Isothiazolyl, Isothiazolidinyl, Morpholinyl, Naphthyridinyl, Oxadiazolyl, 2-Oxoazepinyl, 2-Oxopiperazinyl, 2-Oxopiperidinyl, 2-Oxopyrrolidinyl, Piperidyl, Piperazinyl, Pyridyl, Pyridyl-N-oxid, Chinoxalinyl, Tetrahydrofuryl, Tetrahydroisochinolinyl, Tetrahydrochinolinyl, Thiamorpholinyl, Thiamorpholinylsulfoxid, Thiazolyl, Thiazolinyl, Thienofuryl, Thienothienyl oder Thienyl ist, und
wobei das Aryl Phenyl oder Naphthyl ist,
mit der Maßgabe, dass
R⁶ H ist, und R¹⁰ und R⁷ zusammen genommen werden, um 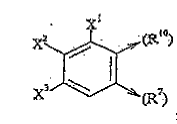 zu bilden, oder
R⁷ =O, -H oder =S ist, und R¹⁰ und R⁶ zusammen genommen werden, um 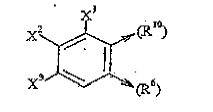 zu bilden,
wobei X¹, X² und X³ jeweils unabhängig H, Halogen. -NO₂, -NCO-R⁸, -CO₂R⁸, -CN oder -CON(R⁸R⁹) sind,
oder ein pharmazeutisch annehmbares Salz davon.

2. Pharmazeutische Zusammensetzung nach Anspruch 1, in der
R¹ 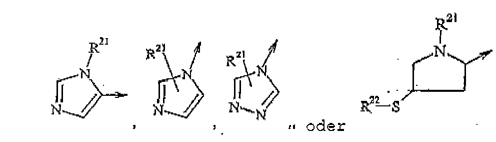 ist, und
X CH(R¹¹)ₙ₃(CH₂)ₙ₄ ist,
oder ein pharmazeutisch annehmbares Salz davon.

3. Pharmazeutische Zusammensetzung nach Anspruch 2, in der
R¹ 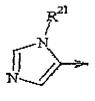 ist,
R⁶ ist H;
n1 1 ist,
R⁷ und R¹⁰ zusammen genommen werden um 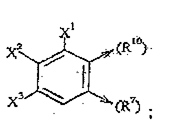 zu bilden,
n3 1 ist, und R¹¹ H ist,
Z eine Bindung ist,
n5 0 ist, und
Y CO ist,
oder ein pharmazeutisch annehmbares Salz davon.

4. Pharmazeutische Zusammensetzung nach Anspruch 2, bei der der Farnesyltransferaseinhibitor eine Verbindung mit der Formel I ist, in der
R¹ 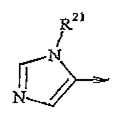 ist,
R⁷ H oder =O ist,
n1 1 ist,
R⁶ und R¹⁰ zusammen genommen werden, um 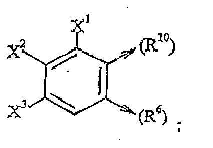 zu bilden,
n3 1 ist, und R¹¹ H ist,
n5 0 ist,
Y CO ist, und
Z eine Bindung ist,
oder ein pharmazeutisch annehmbares Salz davon.

5. Pharmazeutische Zusammensetzung nach Anspruch 1, bei der der Farnesyltransferaseinhibitor
1-(2-(1-(4-Cyanophenylmethyl)imidazol-4-yl)-1-oxoethyl)-1,2-dihydro-4-(2-methoxyphenyl)-imidazo[1,2-c][1,4]benzodiazepin,
9-Brom-1-(2-(1-(4-cyanophenylmethyl)imidazol-4-yl)-1-oxe-eethyl)-1,3-dihydro-4--(2-methoxyphenyl)-imidazo[1,2-c]-[1,4]benzodiazepin,
9-Chlor-1-(2-(1-(4-cyanophenylmethyl)imidazol-4-yl)-1-oxoethyl)-1,2-dihydro-4-(2-methoxyphenyl)-imidazo[1,2-c]-[1,4]benzodiazepin,
10-Brom-1-(2-(1-(4-cyanophenylmethyl)imidazol-4-yl)-1-oxoethyl)-1,2-dihydro-4-(2-methoxyphenyl)imidazo[1,2-c]-[1,4]benzodiazepin,
1-(2-(1-(4-Cyanophenylmethyl)midazol-4-yl)-1-oxoethyl)-1,2-dihydro-8-fluor-4-(2-methoxyphenyl)-imidazo[1,2-c]-[1,4]benzodiazepin
oder ein pharmazeutisch annehmbares Salz davon ist.

6. Pharmazeutische Zusammensetzung nach Anspruch 9, bei der der Farnesyltransferaseinhibitor
1-(2-(1-(4-Cyanophenylmethyl)imidazol-4-yl)-1-oxoethyl)-1,2-dihydro-4-(2-methoxyphenyl)-imidazo[1,2-c][1,4]benzodiazepin,
9-Brom-1-(2-(1-(4-cyanophenylmethyl)imidazol-4-yl)-1-oxo-ethyl)-1,2-dihydro-4-(2-methoxyphenyl)-imidazo[1,2-c]-[1,4]benzodiazepin,
9-Chlor-1-(2-{1-(4-cyanophenylmethyl)imidazol-4-yl)-1-oxoethyl)-1,2-dihydro-4-(2-methoxyphenyl)-imidazo[1,2-c]-[1,4]benzodiazepin,
10-Brom-1-(2-(1-(4-cyanophenylmuthyl)imidazol-4-yl)-1-oxoethyl)-1,2-dihydro-4-(2-methoxyphenyl)-imidazo[1,2-c]-[1,4]benzodiazepin,
1-(2-(1-(4-Cyanophenylmethyl)imidazol-4-yl)-1-oxoethyl)-1,2-dihydro-8-fluor-4-(2-methoxyphenyl)-imidazo[1,2-c]-[1,4]benzodiazepin ist.

7. Pharmazeutische Zusammensetzung nach Anspruch 1, bei der der Farnesyltransferaseinhibitor
5-(2-(1-(4-Cyanophenylmethyl)imidazol-5-yl)-1-oxoethyl)-5,6-dihydro-2-phenyl-1H-imidazo[1,2-a][1,4]benzodiazepin oder ein pharmazeutisch annehmbares Salz davon ist.

8. Pharmazeutische Zusammensetzung nach Anspruch 1, bei der der Farnesyltransferaseinhibitor
1,2-Dihydro-1-(2-(imidazol-1-yl)-1-oxoethyl)-4-(2-methoxyphenyl)imidazo[1,2a][1,4]benzodiazepin
oder ein pharmazeutisch annehmbares Salz davon ist.

9. Pharmazeutische Zusammensetzung nach Anspruch 1, bei der der Farnesyltransferaseinhibitor 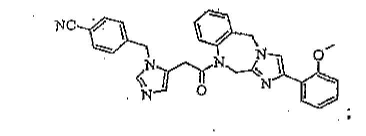 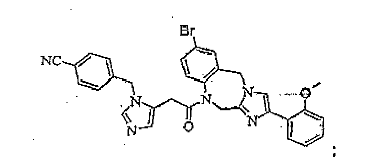 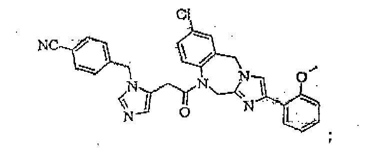 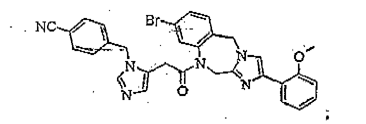 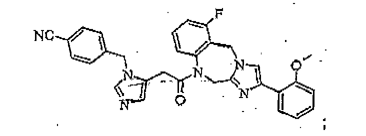 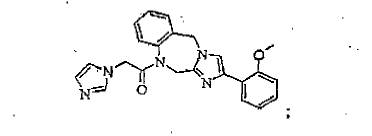 oder 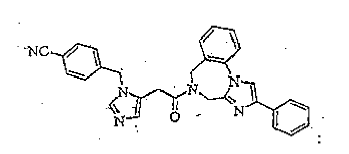 oder ein pharmazeutisch annehmbares Salz davon ist.

10. Pharmazeutische Zusammensetzung nach Anspruch 1, bei der der Farnesyltransferaseinhibitor 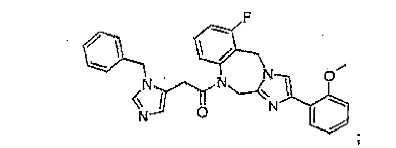 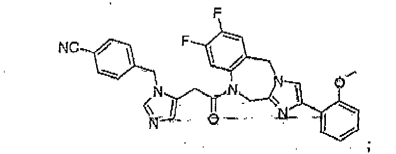 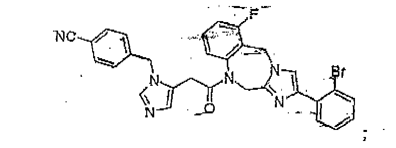 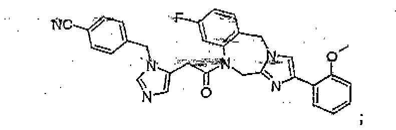 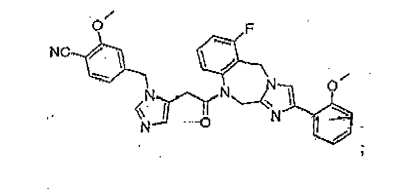 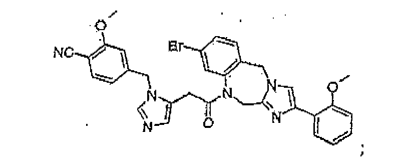 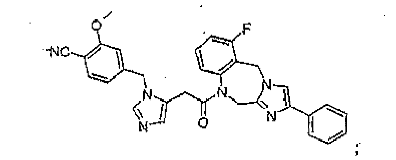 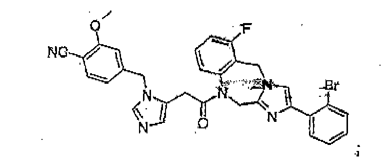 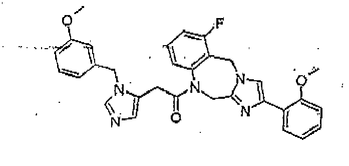 oder 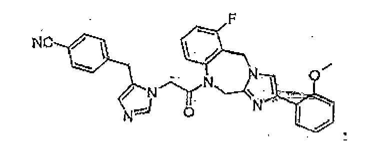 oder ein pharmazeutisch annehmbares Salz davon ist.

11. Pharmazeutische Zusammensetzung nach Anspruch 10, bei der der Farnesyltransferaseinhibitor 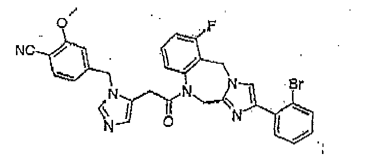 oder ein pharmazeutisch annehmbares Salz davon ist.

12. Pharmazeutische Zusammensetzung nach Anspruch 11, bei der das Anthracyclin Doxorubicin, Daunorubicin, Epirubicin, Idarubicin oder Amrubicin oder ein pharmazeutisch annehmbares Salz davon ist.

13. Pharmazeutische Zusammensetzung nach Anspruch 11, bei der das Anthracyclin Doxorubicin oder ein pharmazeutisch annehmbares Salz davon ist.

14. Pharmazeutische Zusammensetzung nach einem der Ansprüche 1 bis 11, bei der das Anthracyclin Doxorubicin, Daunorubicin, Epirubicin, Idarubicin oder Amrubicin oder ein pharmazeutisch annehmbares Salz des Anthracyclins ist.

15. Pharmazeutische Zusammensetzung nach Anspruch 14, bei der das Anthracyclin Doxorubicin oder ein pharmazeutisch annehmbares Salz davon ist.

16. In-vitro-Verfahren zur Herabsetzung der Proliferationsrate von Nasopharyngealkarzinomzellen, bei dem die Nasopharyngealzellen mit einer pharmazeutischen Zusammensetzung gemäß einem der Ansprüche 12 bis 15 in Kontakt gebracht werden.

17. Verwendung einer pharmazeutischen Zusammensetzung gemäß einem der Ansprüche 12 bis 15 zur Herstellung eines Medikaments zur Behandlung des Nasopharyngealkarzinoms bei einem Patienten.

18. Verwendung einer wirksamen Menge von einem oder mehreren Farnesyltransferaseinhibitoren gemäß Anspruch 1 in Kombination mit einer wirksamen Menge von einem oder mehreren Anthracyclinen zur Herstellung eines Medikaments zur Behandlung von Nasopharyngealkarzinom bei einem Patienten, wobei die wirksame Menge des Farnesyltransferaseinhibitors und des Anthracyclins in Kombination zur Behandlung des Nasopharyngealkarzinoms wirksam sind.

19. Verwendung nach Anspruch 18, bei der der Patient ein Säuger ist.

20. Verwendung nach Anspruch 19, bei der der Patient ein Mensch ist.

21. Verwendung nach Anspruch 20, bei der der Farnesyltransferaseinhibitor und das Anthracyclin im Wesentlichen gleichzeitig verabreicht werden.

22. Pharmazeutischer Kit, der eine Zusammensetzung gemäß einem der Ansprüche 12 bis 15 und Anweisungen zur Verwendung der Zusammensetzung zur Behandlung des Nasopharyngealkarzinoms umfasst.

23. Kit, der a) eine erste Einheitsdosierform, die einen Farnesyltransferaseinhibitor gemäß Anspruch 1 oder ein pharmazeutisch annehmbares Salz davon und einen pharmazeutisch annehmbaren Träger, ein pharmazeutisch annehmbares Vehikel oder Verdünnungsmittel umfasst, b) eine zweite Einheitsdosierform, die ein Anthracyclin oder ein pharmazeutisch annehmbares Salz davon und einen pharmazeutisch annehmbaren Träger, ein pharmazeutisch annehmbares Vehikel oder Verdünnungsmittel umfasst, und c) einen Behälter umfasst.

24. Kit nach Anspruch 23, bei dem der Farnesyltransferaseinhibitor eine Verbindung mit der Formel I oder ein pharmazeutisch annehmbares Salz davon umfasst, und das Anthracyclin Doxorubicin, Daunorubicin, Epirubicin, Idarubicin oder Amrubicin oder ein pharmazeutisch annehmbares Salz davon umfasst.

25. Kit nach Anspruch 24, bei dem der Farnesyltransferaseinhibitor eine Verbindung mit der Formel 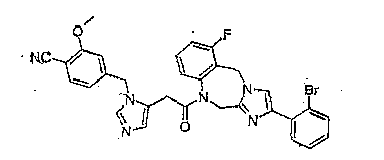 oder ein pharmazeutisch annehmbares Salz davon umfasst, und das Anthracyclin Doxorubicin oder ein pharmazeutisch annehmbares Salz davon umfasst.

## Revendications

1. Composition pharmaceutique comprenant un inhibiteur de farnésyl transférase ou un sel pharmaceutiquement acceptable de celui-ci et une anthracycline, ou un sel pharmaceutiquement acceptable de celle-ci, ledit inhibiteur de farnésyl transférase étant conforme à la formule I : 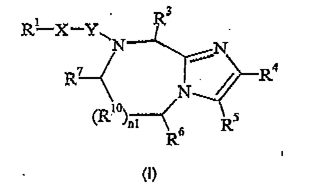 dans laquelle :
n1 est 1 ;
X est, indépendamment pour chaque occurrence, (CHR¹¹)ₙ₃(CH₂)ₙ₉Z(CH₂)ₙ₅ ;
Z est une liaison ;
n3 est, indépendamment pour chaque occurrence, 0 ou 1 :
n4 et n5 sont chacun, indépendamment pour chaque occurrence, 0, 1, 2, ou 3 ;
Y est, indépendamment pour chaque occurrence, CO ;
R₁ est 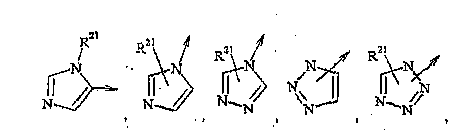 ou 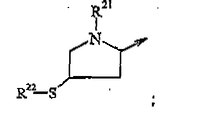
R² et R¹² sont chacun, indépendamment pour chaque occurrence, H ou une fraction facultativement substituée choisie dans le groupe constitué par alkyle en C₁₋₆ et aryle, ladite fraction facultativement substituée étant facultativement substituée par un de R⁶ ou R³⁰ ou davantage ;
R¹¹ est, indépendamment pour chaque occurrence, H ;
R³ est, indépendamment pour chaque occurrence, H ou une fraction facultativement substituée choisie dans le groupe constitué par alkyle en C₁₋₆, alcényle en C₂₋₆, alcynyle en C₂₋₆, cycloalkyle en C₃₋₆, cycloalkyl en C₃₋₆-alkyle en C₁₋₆, cycloalcényle en C₅₋₇, cycloalcényl en C₅₋₇-alkyle en C₁₋₆, aryle, aryl-alkyle en C₁₋₆, hétérocyclyle et hétérocyclyl-alkyle en C₁₋₆, ladite fraction facultativement substituée étant facultativement substituée par un R³⁰ ou davantage ;
R⁴ est, indépendamment pour chaque occurrence, une fraction phényle facultativement substituée, ladite fraction phényle facultativement substituée étant facultativement substituée par un R³⁰ ou davantage, chaque substituant précité étant indépendamment choisi ;
R⁵ est, indépendamment pour chaque occurrence, H ;
R⁶ est, indépendamment pour chaque occurrence, H ou une fraction facultativement substituée choisie dans le groupe constitué par alkyle en C₁₋₆, alcényle en C₂₋₆, cycloalkyle en C₃₋₆, cycloalkyl en C₃₋₆-alkyle en C₁₋₆, cycloalcényle en C₅₋₇, cycloalcényl en C₅₋₇-alkyle en C₁₋₆, aryle, aryl-alkyle en C₁₋₆, hétérocyclyle et hétérocyclyl-alkyle en C₁₋₆, ladite fraction facultativement substituée étant facultativement substituée par un ou plusieurs substituants choisis chacun indépendamment dans le groupe constitué par OH, alkyle en C₁₋₆, alkoxy en C₁₋₆, -N(R⁸R⁹), -COOH, -CON(R⁸R⁹), et halo, R⁸ et R⁹ étant chacun, indépendamment pour chaque occurrence, H, alkyle en C₁₋₆, alcényle en C₂₋₆, alcynyle en C₂₋₆, aryle ou aryl-alkyle en C₁₋₆ ;
R⁷ est, indépendamment pour chaque occurrence, H, =O, =S, ou une fraction facultativement substituée choisie dans le groupe constitué par alkyle en C₁₋₆, alcényle en C₂₋₆, cycloalkyle en C₃₋₆, cycloalkyl en C₃₋₆-alkyle en C₁₋₆, cycloalcényle en C₅₋₇, cycloalcényl en C₅₋₇-alkyle en C₁₋₆, aryle, aryl-alkyle en C₁₋₆, hétérocyclyle et hétérocyclyl-alkyle en C₂₋₆, ladite fraction facultativement substituée étant facultativement substituée par un ou plusieurs substituants choisis chacun indépendamment dans le groupe constitué par OH, alkyle en C₁₋₆, alkoxy en C₁₋₆, -N(R⁸R⁹), -COOH, -CON(R⁸R⁹) et halo ;
R¹⁰ est C ;
R²¹ est, indépendamment pour chaque occurrence, H ou une fraction facultativement substituée choisie dans le groupe constitué par alkyle en C₁₋₆ et aryl-alkyle en C₁₋₆, ladite fraction facultativement substituée étant facultativement substituée par un substituant ou davantage choisi chacun indépendamment dans le groupe constitué par R⁸ et R³⁰ _{;}
R¹² représente H, alkylthio en C₁₋₆, cycloalkylthio en C₃₋₆, R⁸-CO-, ou un substituant conforme à la formule 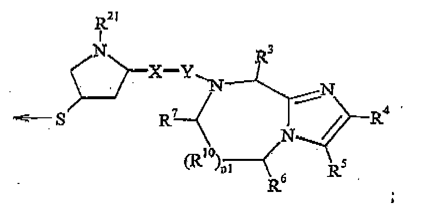
R²⁴ et R²⁵ sont chacun, indépendamment pour chaque occurrence, H, alkyle en C₁₋₆, ou aryl-alkyle en C₁₋₆ ;
R³⁰ est, indépendamment pour chaque occurrence, alkyle en C₁₋₆, -O-R⁶, -S(O)ₙ₆R⁸, -S(O)ₙ₇N(R⁸R⁹), -N(R⁸R⁹), -CN,
-NO₂, -CO₂R⁸, -CON(R⁸R⁹), -NCO-R⁸ ou halogène ;
n6 et n7 sont chacun, indépendamment, pour chaque occurrence, 0, 1 ou 2 ;
ledit hétérocyclyle étant azépinyle, benzimidazolyle, benzisoxazolyle, benzofurazanyle, benzopyranyle, benzothiopyranyle, benzofuryle, benzothiazolyle, benzothiényle, benzoxazolyle, chromanyle, cinnolinyle, dihydrobenzofuryle, dihydrobenzothiényle, dihydrobenzothiopyranyle, dihydrobenzothio-pyranyl sulfone, furyle, imidazolidinyle, imidazolinyle, imidazolyle, indolinyle, indolyle, isochromanyle, isoindolinyle, isoquinolinyle, isothiazolidinyle, isothiazolyle, isothiazolidinyle, morpholinyle, naphtyridinyle, oxadiazolyle, 2-oxoazépinyle, 2-oxopipérazinyle, 2-oxopipéridinyle, 2-oxopyrrolidinyle, pipéridyle, pipérazinyle, pyridyle, pyridyl N-oxyde, quinoxalinyle, tétrahydrofuryle, tétrahydroisoquinolinyle, tétrahydroquinolinyle, thiamorpholinyle, thiamorpholinyl sulfoxyde, thiazolyle, thiazolinyle, thiénofuryle, thiénothiényle ou thiényle, et
ledit aryle étant phényle ou naphtyle ;
à la condition que :
R⁶ est H, et R¹⁰ et R⁷ sont pris chacun pour former 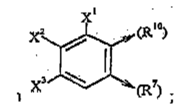 ou ;
R⁷ est =O, -H, ou =S, et R¹⁰ et R⁶ sont pris conjointement pour former 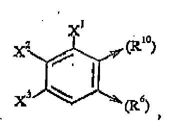
où X¹, X² et X³ sont chacun, indépendamment, H, halogène, - NO₂, -NOCO-R⁸, -CO₂R⁸, -CN ou -CON(R⁸R⁹) ;
ou un sel pharmaceutiquement acceptable de celui-ci.

2. Composition pharmaceutique selon la revendication 1, dans laquelle :
R¹ est 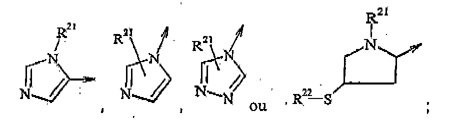 et
X est CH(R¹¹)ₙ₃(CH₂)ₙ₄ ;
ou un sel pharmaceutiquement acceptable de celui-ci.

3. Composition pharmaceutique selon la revendication 2, dans laquelle :
R¹ est 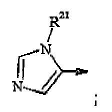
R⁶ est H ;
n1 est 1 ;
R⁷ et R¹⁰ sont pris conjointement pour former 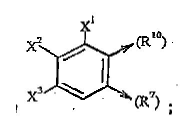
n3 est 1 et R¹¹ est H ;
Z est une liaison ;
n5 est 0 ; et
Y est CO ;
ou un sel pharmaceutiquement acceptable de celui-ci.

4. Composition pharmaceutique selon la revendication 2, dans laquelle ledit inhibiteur de farnésyl transférase est un composé de formule I, dans laquelle :
R¹ est 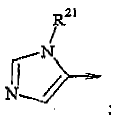
R⁷ est H ou =O :
n1 est 1 ;
R⁶ et R¹⁰ sont pris conjointement pour former 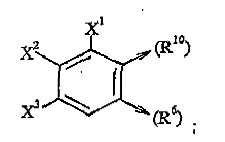
n3 est 1 et R¹¹ est H ;
n5 est 0 ;
Y est CO ; et
Z est une liaison ;
ou un sel pharmaceutiquement acceptable de celui-ci.

5. Composition pharmaceutique selon la revendication 1, dans laquelle ledit inhibiteur de farnésyl transférase est :
- la 1-(2-(1-(4-cyanophénylméthyl)imidazol-4-yl)-1-oxoéthyl)-1,2-dihydro-4-(2-méthoxyphényl)-imidazo[1,2-c][1,4]benzodiazépine ;
- la 9-bromo-1-(2-(1-(4-cyanophénylméthyl)imidazol-4-yl)-1-oxoéthyl)-1,2-dihydro-4-(2-méthoxyphényl)-imidazo[1,2-c][1,4]benzodiazépine ;
- la 9-chloro-1-(2-(1-(4-cyanophénylméthyl)imidazol-4-yl)-1-oxoéthyl)-1,2-dihydro-4-(2-méthoxyphényl)-imidazo[1,2-o][1,4]benzodiazépine ;
- la 10-bromo-1-(2-(1-(4-cyanophénylméthyl)imidazol(-4-yl)-1-oxoéthyl)-1,2-dihydro-4-(2-méthoxyphényl)-imidazo[1,2-c][1,4]benzodiazépine ;
- la 1-(2-(1-(4-cyanophénylméthyl)imidazol-4-yl)-1-oxoéthyl)-1,2-dihydro-8-fluoro-4-(2-méthoxyphényl)-imidazo[1,2-c][1,4]benzodiazépine ; ou
- un sel pharmaceutiquement acceptable de celles-ci.

6. Composition pharmaceutique selon la revendication 9, dans laquelle ledit inhibiteur de farnésyl transférase est :
- la 1-(2-(1-(4-cyanophénylinéthyl)imidazol-4-yl)-1-oxoéthyl)-1,2-dihydro-4-(2-méthoxyphényl)-imidazo[1,2-c][1,4]benzodiazépine ;
- la 9-bromo-1-(2-(1-(4-cyanophénylméthyl)imidazol-4-yl)-1-oxoéthyl)-1,2-dihydro-4-(2-méthoxyphényl)-imidazo[1,2-c][1,4]benzodiazépine ;
- la 9-chloro-1-(2-(1-(4-cyanophénylméthyl)imidazol-4-yl)-1-oxoéthyl)-1,2-dihydro-4-(2-méthoxyphényl)-imidazo[1,2-c][1,4]benzodiazépine ;
- la 10-bromo-1-(2-(1-(4-cyanophénylméthyl)imidazol-4-yl)-1-oxoéthyl)-1,2-dihydro-4-(2-méthoxyphényl)-imidazo[1,2-c][1,4]benzodiazépine ;
- la 1-(2-(1-(4-cyanophénylméthyl)imidazol-4-yl)-1-oxoéthyl)-1,2-dihydro-8-flucro-4-(2-méthoxyphényl)-imidazo[1,2-c][1,4]benzodiazépine.

7. Composition pharmaceutique selon la revendication 1, dans laquelle ledit inhibiteur de farnésyl transférase est :
- la 5-(2-(1-(4-cyanophénylméthyl)-imidazol-5-yl)-1-oxo-éthyl)-5,6-dihydro-2-phényl-1H-imidazo[1,2-a][1,4]benzodiazépine ;
ou un sel pharmaceutiquement acceptable de celle-ci.

8. Composition pharmaceutique selon la revendication 1, dans laquelle ledit inhibiteur de farnésyl transférase est :
- la 1,2-dihydro-l-(2-(imidazol-1-yl)-1-oxoéthyl)-4-(2-méthoxyphényl)imidazo[1,2a][1,4]benzodiazépine ;
ou un sel pharmaceutiquement acceptable de celle-ci.

9. Composition pharmaceutique selon la revendication 1, dans laquelle ledit inhibiteur de farnésyl transférase est : 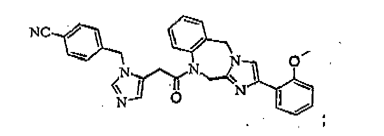 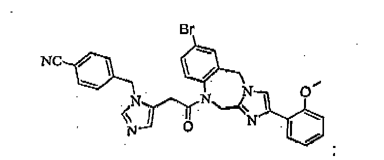 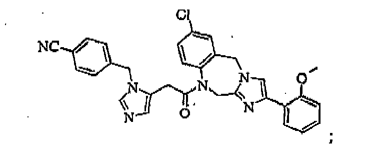 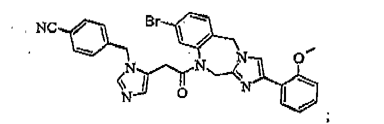 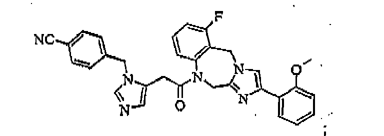 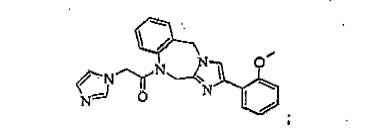 ou 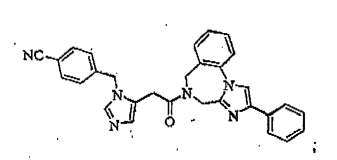 ou un sel pharmaceutiquement acceptable de celle-ci.

10. Composition pharmaceutique selon la revendication 1, dans laquelle ledit inhibiteur de farnésyl transférase est : 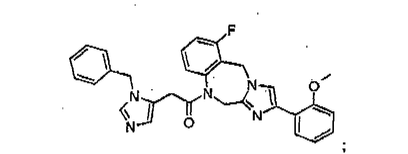 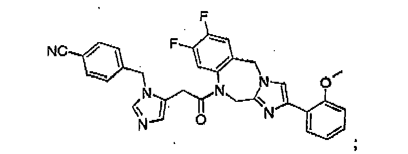 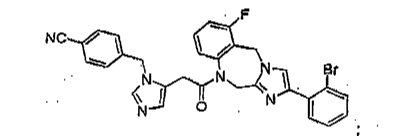 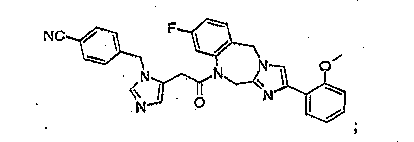 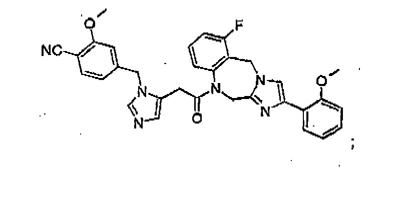 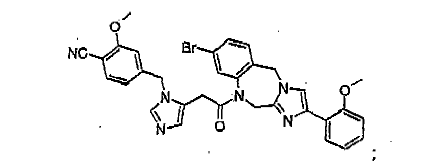 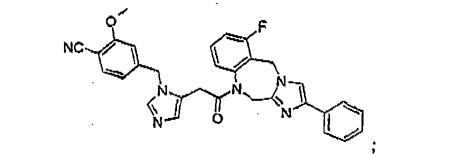 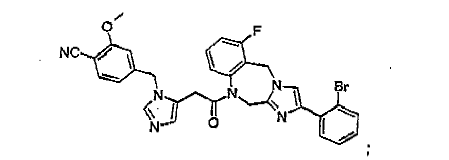 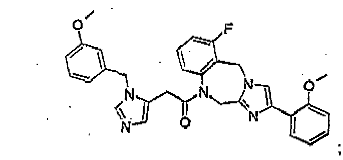 or 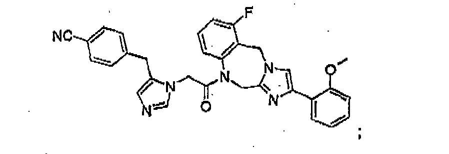 ou un sel pharmaceutiquement acceptable de celui-ci.

11. Composition pharmaceutique selon la revendication 10, dans laquelle ledit inhibiteur de farnésyl transférase est : 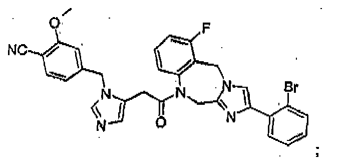 ou un sel pharmaceutiquement acceptable de celui-ci.

12. Composition pharmaceutique selon la revendication 11, dans laquelle ladite anthracycline est la doxorubicine, la daunorubicine, l'épirubicine, l'idarubicine, ou l'amrubucine, ou un sel pharmaceutiquement acceptable de celles-ci.

13. Composition pharmaceutique selon la revendication 11, dans laquelle ladite anthracycline est la doxorubicine ou un sel pharmaceutiquement acceptable de celle-ci.

14. Composition pharmaceutique selon l'une quelconque des revendications 1-11, dans laquelle ladite anthracycline est la doxorubicine, la daunorubicine, l'épirubicine, l'idarubicine ou l'amrubucine, ou un sel pharmaceutiquement acceptable de ladite anthracycline.

15. Composition pharmaceutique selon la revendication 14, dans laquelle ladite anthracycline est la doxorubicine ou un sel pharmaceutiquement acceptable de celle-ci.

16. Procédé in vitro de diminution de la vitesse de prolifération de cellules de carcinome rhinopharyngé, ledit procédé comprenant la mise en contact desdites cellules rhinopharyngées avec une composition pharmaceutique telle que définie à l'une quelconque des revendications 12 à 15.

17. Utilisation d'une composition pharmaceutique telle que définie à l'une quelconque des revendications 12 à 15 dans la préparation d'un médicament pour le traitement du carcinome rhinopharyngé dans un patient.

18. Utilisation d'une quantité efficace d'un ou plusieurs inhibiteurs de farnésyl transférase tels que définis dans la revendication 1 en combinaison avec une quantité efficace d'une anthracycline ou davantage, dans la préparation d'un médicament pour le traitement du carcinome rhinopharyngé dans un patient, ladite quantité efficace dudit inhibiteur de farnésyl transférase et de ladite anthracycline étant efficaces en combinaison pour traiter ledit carcinome rhinopharyngé.

19. Utilisation selon la revendication 18, dans laquelle ledit patient est un mammifère.

20. Utilisation selon la revendication 19, dans laquelle ledit patient est un être humain.

21. Utilisation selon la revendication 20, dans laquelle ledit inhibiteur de farnésyl transférase et ladite anthracycline sont administrés sensiblement simultanément.

22. Coffret pharmaceutique comprenant une composition telle que définie à l'une quelconque des revendications 12 à 15 et des instructions pour une utilisation de ladite composition pour le traitement du carcinome rhinopharyngé.

23. Coffret comprenant : a) une première forme posologique unitaire comprenant un inhibiteur de farnésyl transférase tel que défini à la revendication 1 ou un sel pharmaceutiquement acceptable de celui-ci et un support, véhicule ou diluant pharmaceutiquement acceptable ; b) une seconde forme posologique unitaire comprenant une anthracycline, ou un sel pharmaceutiquement acceptable de celle-ci et un support, véhicule ou diluant pharmaceutiquement acceptable ; et c) un récipient.

24. Coffret selon la revendication 23, dans lequel ledit inhibiteur de farnésyl transférase comprend un composé selon la formule I ou un sel pharmaceutiquement acceptable de celui-ci, et ladite anthracycline comprend la doxorubicine, la daunorubicine, l'épirubicine, l'idarubicine ou l'amrubicine ou un sel pharmaceutiquement acceptable de celles-ci.

25. Coffret selon la revendication 24, dans lequel ledit inhibiteur de farnésyl transférase comprend un composé selon la formule : 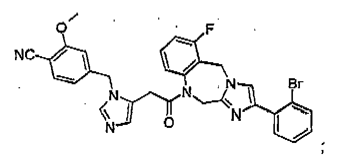 ou un sel pharmaceutiquement acceptable de celui-ci, et ladite anthracycline comprend la doxorubicine ou un sel pharmaceutiquement acceptable de celle-ci.
